(19) Europäisches Patentamt
European Patent Office
Office européen des brevets

(11) EP 3 975 992 B1

(12) # EUROPEAN PATENT SPECIFICATION

(45) Date of publication and mention of the grant of the patent:
**19.07.2023 Bulletin 2023/29**

(21) Application number: **20731805.6**

(22) Date of filing: **28.05.2020**

(51) International Patent Classification (IPC):
***A61P 3/10*** *(2006.01)* ***A61P 9/00*** *(2006.01)*
***A61P 17/00*** *(2006.01)* ***A61P 25/00*** *(2006.01)*
***A61P 35/00*** *(2006.01)* ***A61K 9/08*** *(2006.01)*
***A61K 31/353*** *(2006.01)* ***A61K 36/82*** *(2006.01)*
***A61K 47/18*** *(2017.01)* ***A61K 47/20*** *(2006.01)*
***A23F 3/14*** *(2006.01)* ***A61Q 19/08*** *(2006.01)*
***A61K 8/41*** *(2006.01)* ***A61K 8/9789*** *(2017.01)*
***A61K 31/14*** *(2006.01)*

(52) Cooperative Patent Classification (CPC):
(C-Sets available)
**A61K 9/0014; A23F 3/14; A61K 8/416; A61K 8/9789; A61K 9/08; A61K 31/14; A61K 31/353; A61K 36/82; A61K 47/186; A61K 47/20; A61P 3/10; A61P 9/00; A61P 17/00; A61P 25/00; A61P 35/00;** (Cont.)

(86) International application number:
**PCT/EP2020/064876**

(87) International publication number:
**WO 2020/239929 (03.12.2020 Gazette 2020/49)**

(54) **GREEN TEA CATECHINS EUTECTIC SYSTEM**

EUTEKTISCHE SYSTEME MIT GRÜNTEECATECHINEN

SYSTÈME EUTECTIQUE DE CATÉCHINES DE THÉ VERT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.05.2019 EP 19305692**

(43) Date of publication of application:
**06.04.2022 Bulletin 2022/14**

(73) Proprietors:
- **ASSISTANCE PUBLIQUE-HOPITAUX DE PARIS 75012 Paris 12 (FR)**
- **Université Paris-Saclay 91190 Gif-sur-Yvette (FR)**

(72) Inventors:
- **DO, Bernard 92130 ISSY-LES-MOULINEAUX (FR)**
- **PAUL, Muriel 77144 MONTEVRAIN (FR)**
- **ASTIER, Alain 75010 PARIS (FR)**
- **THIRION, Olivier 91200 ATHIS-MONS (FR)**

(74) Representative: **IPAZ
Bâtiment Platon
Parc Les Algorithmes
91190 Saint-Aubin (FR)**

(56) References cited:
**EP-A1- 2 263 481 WO-A1-2014/055830**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)
**A61Q 19/08**

C-Sets
**A61K 31/14, A61K 2300/00;**
**A61K 31/353, A61K 2300/00**

## Description

The present invention concerns a green tea catechins eutectic system, a pharmaceutical, a dermo-cosmetic or a nutraceutical composition comprising said green tea catechins eutectic system.

Green tea, which makes up around 20 % of tea production worldwide, is consumed most often in China, Korea, and Japan. It is considered as one of the most promising nutrients for the prevention and/or treatment of many diseases. Over the past 15-20 years, a number of research studies have been conducted to determine what health benefits can be attributed to consumption of green tea and its extracts. This research has shown that green tea has a variety of potential health benefits, including anticarcinogenic *(*Crit. Rev. Food Sci. Nutr. 2015, 55, 792-80), anti-inflammatory, antimicrobial, and antioxidant properties, and benefits in cardiovascular disease (Curr. Opin. Clin. Nutr. Metab. Care 2008, 11, 758-765*)* and oral health (Geriatr. Gerontol. Int. 2014, 14, 238-250*;* Int. J. Dent. Hyg. 2011, 9, 110-116*;* ISRN Prev. Med. 2012, 2013, 975148*).*

The components of green tea that are the most relevant medically are polyphenols, with flavonoids being the most important. The most pertinent flavonoids are catechins, which make up 80 % - 90 % of the flavonoids, and approximately 40 % of the water-soluble solids in green tea (J. Agric. Food Chem. 2010, 58, 1296-1304). Green tea contains four main catechins: (-)-epicatechins (EC), (-)-epigallocatechin (EGC), (-)-epicatechin-3-gallate (ECG) and (-)-epigallocatechin-3-gallate (EGCG). The most abundant of these in green tea is EGCG, which represents around 59 % of total catechins and is the responsible polyphenol for the health benefits produced by green tea. The next most abundant is EGC (around 19 %), then ECG (around 14 %) and EC (around 6 %) (Int. Res. J. Pharm. 2012, 3, 139-148). The content of EGCG in green tea extract varies according to the season, the type of leaves and the part of the plant used. The dry extracts of green tea contain about 35 % of polyphenols, 30 % of which are due to catechins, which themselves contain 55 - 80 % of EGCG.

The health benefits of green tea or green tea extracts (GTE) depend on its bioavailability after consumption. Studies on bioavailability are often conducted collecting specimens at timed intervals (after ingestion). Various studies have been conducted using prepared green tea beverages, ingested green tea extract or ingestion of specific catechins. These studies have suggested that levels of detected epicatechins (EC) and epicatechin-3-gallate (ECG) are too low to be of any therapeutic value, so most research considers only EGC and EGCG, mostly EGCG (Front. Microbiol. 2014, 5, 434).

EGCG is currently available as herbal drugs or green tea extracts *(monograph 1433, current European Pharmacopoeia; monograph 2668, Pharmeuropa June 2017*)*,* isolated or not. Tea brewages and the capsules or tablets co-formulated with vitamins account for the main paths for EGCG intake.

Despite the fact that EGCG exhibits remarkable in vitro-antioxidant properties, only limited effects have been observed in animals and human epidemiological studies. After oral administration, the bioavailability of EGCG was found to be very low in humans, resulting in plasma concentrations 5 to 50 times less than concentration shown to exert biological activities in *in vitro* systems (Chow et al., Clin Cancer Res 11 (2005) 4627-4633).

Indeed, like the other green tea catechins, EGCG suffers from poor solubility and low skin permeability.

Poor biopharmaceutical properties of EGCG are mainly due to its relative low solubility in water, low chemical stability, low permeability and a short plasma half-life.

Indeed, at normal condition EGCG is soluble only at a rate of 4.6 g/L (10 mM). The quantity of soluble EGCG is far from sufficient for developing a pharmaceutical product, which needs a high concentration of active ingredient in a small volume.

In addition, EGCG is prompt to react by auto-oxidation and thus exhibits very low chemical stability. Auto-oxidation of EGCG yields reactive oxygen species capable of inducing polymerization and decomposition of EGCG. The resulting quinone derivatives, dimers or oligomers have much lower intestinal absorption rates.

In general, its immediate environment, such as the presence of water, pH, temperature, oxygen content, antioxidant levels and metal ions, even in trace amounts (iron, zinc, copper, aluminium) directly affects its stability (Krupkova et al., Journal of Nutritional Biochemistry 37 (2016) 1-12).

By the way, the low bioavailability is also due in part to its interactions with nutrients present, including co-formulated excipients. Indeed, studies have shown that commonly sparingly soluble components of the capsule and those of the gelatin shell itself can combine with EGCG after disintegration and form even less soluble complexes which impede absorption.

Above-mentioned drawbacks also prevent EGCG to be used in skin care and oral care products. Indeed, in addition to its poor skin permeability and low solubility in water, EGCG is very susceptible to light irradiation and pH superior to 5.0 (J. Pharm. Biomed. Anal. (2011) 56: 692-697*;*). EGCG was found to decompose by 70 %, after 1-h ultra violet irradiation. Because EGCG is a very useful ingredient for protection of the skin against the damaging effects of solar radiation, its stabilization under exposure to ultra violet irradiation is of paramount importance for the preparation of effective dermatological products containing EGCG. With regard to the use of botanical products in oral care, it should be noted that the pH of toothpastes, for example, could vary from 7 to 10, depending on the additives they contain.

Indeed, toothpaste is not produced at low pH to avoid dissolving the enamel and dentin mineral and accelerating the corrosion of dental prostheses. However, these pH conditions are hardly compatible with the chemical stability of the green tea catechin compounds (Chem. Pharm. Bull. 62(4) 328-335 (2014)).

In order to improve the solubility and stability of EGCG in the context of topical administrations, a great number of technologies have been tried and proposed. However, EGCG formulations described to this day still cannot completely meet the requirements for clinical development of EGCG.

US20140088030 described a method for producing O-o-glucoside derivatives of phenolic compounds (such as EGCG), by incubating sucrose and a glucansucrase from *Leuconostoc* species with the phenolic compounds. However, this method involves structural changes of EGCG. These changes may impair other properties of EGCG.

The mushrooming area of nanotechnology has led to the development of potential chemotherapy involving nanoparticles (NPs). Various particles (e.g. gold) can be used to deliver compounds to specific areas of the body. Research using EGCG and nanoparticles has already begun using a number of delivery approaches. These include: coating an NP, such as gold, with EGCG; use of encapsulated (in liposomes or polymeric NPs) EGCG in NPs along with anticancer drugs, outer ligands that will bind to specific targets, or outer polymers that will enhance the intestinal absorption of EGCG (Nutrients 2016, 8, E307; Journal of Nutritional Biochemistry 37 (2016) 1-12). However, changes in release rates and kinetics could make the EGCG biopharmaceutical phase even more complex and far less predictable. By the way, for equivalent doses administered, it is possible that the use of a form gradually releasing EGCG, which is therefore present at smaller doses in the gastrointestinal tract, would expose it more to enzymatic degradations and bacterial metabolisms than an immediate release form.

Krupkova et al. (Journal of Nutritional Biochemistry 37 (2016) 1-12) described a solution formulation of green tee catechins (GTC) comprising 1.09 mM of a GTC mixture, 0.15 g/mL of sucrose and 2 mg/mL of citric acid. However, a 6-month assessment revealed that addition of sucrose had little effect on the stability of GTC, whereas addition of citric acid destabilized GTC.

EP17306623 describes a stable pharmaceutical aqueous solution which increases nearly 20 times EGCG concentration in aqueous solution in the presence of a disaccharide without resorting to temperature and organic solvents. However, given the high content of disaccharide in these formulations, these latter may be less suitable for the use in topically or externally applied products like the use for oral health.

WO 2014/055830 A1 discloses two liquid nutritional compositions (comparative examples 5 and 6) which comprise among other ingredients EGCG-containing Green Tea Extract, choline chloride and water. The target amount of EGCG is about 738 mg/L (comparative example 5) or 1055 mg/L (comparative example 6).

Therefore, there is still a need in the art to provide a new technical solution to improve EGCG water solubility, stability and bioavailability and to provide a new EGCG composition having improved EGCG water solubility, stability and bioavailability and suitable for use in pharmaceutical, dermo-cosmetic, or nutraceutical products.

The Inventor has developed a liquid eutectic system of EGCG which is formed by EGCG, a choline salt and an aprotic or protic polar solvent, said system being miscible with water. Against all odds, when this eutectic system of EGCG is mixed with water at room temperature, EGCG water solubility can reach 195.5 mg/mL, that is about 40 times higher than intrinsic solubility of EGCG (4.6 mg/mL) in water at room temperature.

The experimental results displayed below in Examples also show that said eutectic system of EGCG is able to efficiently protect EGCG from auto-oxidation, hydrolysis and photolysis than a simple water solution containing uniquely EGCG at the same concentration.

Biological tests carried out with compositions comprising said eutectic system of EGCG show that this system also displays better biological properties, in terms of skin permeability and bioavailability, compared to commercially available EGCG formulation.

Moreover, *in vivo* study suggests that after an important dilution in an appropriate co-solvent, such as after la dilution of 20 times, and even in digestive tube, the eutectic system of the present invention can still be conserved.

The present invention concerns a stable liquid eutectic system comprising :

- an active ingredient chosen from catechins,
- a choline salt,
- a polar protic solvent or aprotic polar solvent which is pharmaceutically, dermo-cosmetically or nutraceutically acceptable,

  wherein the molar ratio between the active ingredient, the choline salt, and the solvent is 0.25-3.5:1:5-75, and wherein the concentration of the active ingredient is at least 100 mg/mL.

By the term "eutectic system" is referred to a system obtained after the melting at a specific temperature of two or more components in a specific range of molar ratio. In a eutectic system, the components melt and solidify at a same temperature which is lower than melting temperature of any other composition made up of the same components.

The eutectic system of the present invention is formed at ambient temperature, that is to say from 15 to 35 °C, particularly from 15 to 29°C, and is liquid at ambient temperature.

Said eutectic system provided by the invention allows to significantly improve water solubility of a compound of class of flavonoids, in particular a catechin, an anthocyanin or a procyanidin, which often has a poor water solubility and low bioavailability.

It has been observed by the inventors that EGCG and small amounts of an aprotic or protic polar solvent such as dimethyl sulfoxide, dimethylacetamide, or water, and a choline salt, e.g. chloride, particularly when present in a certain molar ratio range, become entirely liquid at ambient temperature. While the solubility of EGCG in DMSO or water does generally not exceed respectively 25 mg/mL and 4.6 mg/mL at ambient temperature, the liquid eutectic system of the present invention contains very high concentration of EGCG and at minimum improves by a factor of 40 the solubility of EGCG under the same temperature conditions.

Without being bound by any theory it is believed that due to reciprocal interactions involving the formation of hydrogen bonds, a eutectic system, e.g. between EGCG / choline salt / aprotic or protic polar solvent, is formed which makes it possible to substantially increase EGCG solubility in an aprotic or protic polar solvent. It is also believed that hydrogen bonds formed between EGCG, DMSO and choline chloride may prevent the ester function of EGCG from being exposed to hydrolysis.

Because of high structure similarity, an anthocyanin, a procyanidin or other catechin can also form a deep eutectic system with a choline salt and an aprotic or protic polar solvent, that is to say a eutectic system formed at ambient temperature.

The appropriate molar ratio between the three components of the eutectic system of the invention (an active ingredient, a choline salt, an aprotic or protic polar solvent) is that for 1 molar of the choline salt in said system, there are from 0.25 to 3.5 molar of the active ingredient and from 5 to 75 molar of the aprotic or protic polar solvent.

In a preferred embodiment, the present invention concerns a stable liquid eutectic system comprising :

- an active ingredient chosen from catechins,
- a choline salt,
- a polar protic solvent or aprotic polar solvent which is pharmaceutically, dermo-cosmetically or nutraceutically acceptable,

wherein the molar ratio between the active ingredient, the choline salt, and the solvent is 0.25-1.5:1:5-50, and wherein the concentration of the active ingredient is at least 100 mg/mL.

In another preferred embodiment, the molar ratio between the active ingredient, the choline salt, and the polar solvent is 1-1.5:1:6-25, that is to say for 1 molar of the choline salt in said system, there are from 1 to 1.5 molar of active ingredient and from 6 to 25 molar of polar solvent.

In another preferred embodiment, the molar ratio between the active ingredient, the choline salt, and the polar solvent is 0.25-1.3:1:5-50, that is to say for 1 molar of the choline salt in said system, there are from 0.25 to 1.3 molar of active ingredient and from 5 to 50 molar of polar solvent.

In another preferred embodiment, the molar ratio between the active ingredient, the choline salt, and the polar solvent is 0.25-3.5:1:35-75, that is to say for 1 molar of the choline salt in said system, there are from 0.25 to 3.5 molar of active ingredient and from 35 to 75 molar of polar solvent.

According to a particular embodiment, the present invention concerns a stable liquid eutectic system consisting of:

- an active ingredient chosen from catechins,
- a choline salt,
- a polar protic solvent or aprotic polar solvent which is pharmaceutically, dermo-cosmetically or nutraceutically acceptable,

  wherein the molar ratio between the active ingredient, the choline salt, and the solvent is 0.25-3.5:1:5-75 and wherein the concentration of the active ingredient is at least 100 mg/mL.

According to a particular embodiment, the present invention concerns a stable liquid eutectic system consisting of:

- an active ingredient chosen from catechins,
- a choline salt,
- a polar protic solvent or aprotic polar solvent which is pharmaceutically, dermo-cosmetically or nutraceutically acceptable,

wherein the molar ratio between the active ingredient, the choline salt, and the solvent is 0.25-1.5:1:5-50 and wherein the concentration of the active ingredient is at least 100 mg/mL.

As used herein, the term "catechins" is referred to catechins, epicatechins, gallocatechin, epigallocatechin, particularly (-)-epicatechin gallate (ECG) and (-)-epigallocatechin gallate (EGCG).

In one embodiment of the stable liquid eutectic system, the active ingredient is green tea catechins, particularly chosen from epicatechins, epigallocatechin, epicatechin-3-gallate, epigallocatechin gallate or a mixture thereof.

As used herein, the term "epigallocatechin gallate" means molecular EGCG, or natural EGCG purified from any part of leaves of *Camellia sinensis* picked at any seasons.

Preferably, any extract of EGCG having a content of at least 40%, preferably at least 50%, more preferably at least 60%, more preferably at least 70%, more preferably et least 80%, more preferably at least 90 %, particularly at least 95 %, more particularly at least 97 %, still more particularly at least 98% by weight of EGCG is suitable for preparing the eutectic system of the present invention.

In some embodiments, the extract of EGCG used for preparing a eutectic system of the present invention can be an extract of green tea containing caffeine or a decaffeinated extract of green tea.

Preferably, the extract of EGCG used for preparing a eutectic system of the present invention satisfies the requirements specified in concerned current European pharmacopoeia (*monographs 1433 and* 2668, *current European Pharmacopoeia*).

When used herein, the term "anthocyanin" is referred to a glycosylated form of an anthocyanidin, wherein the sugar is in particular chosen from glucose, arabinose, galactose, or dimers thereof and said anthocyanidin is in particular chosen from cyanidin, delphinidin, pelargonidin, peonidin, malvidin, and petunidin.

Particularly, said anthocyanin can be represented by the following formula

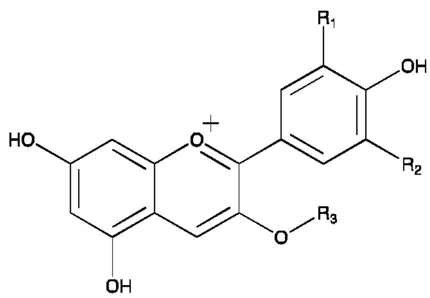

wherein $R_1$ and $R_2$ are independently chosen from -H, -OH, $-OCH_3$; $R_3$ is chosen from glucose, arabinose, galactose, or dimers thereof.

When used herein, the term "dimer" is referred to a sugar formed by two identical monomers mentioned before or by two different monomers mentioned before.

The term "procyanidin" used herein means an oligomeric compound formed from catechin and epicatechin.

In the eutectic system of the invention, the choline salt may include choline chloride, choline hydroxide, choline tartrate, choline dihydrogen citrate, choline acetate, and choline sulphate.

In the eutectic system of the invention, polar solvent can be an aprotic polar solvent or a protic polar solvent. The aprotic solvent can be chosen from the classes of solvents of sulfoxides, ketones, esters, amides. Preferably, the aprotic solvent is chosen from dimethyl sulfoxide (DMSO) or dimethylacetamide.

The polar protic solvent can be chosen from water, ethanol, propylene glycol, such as PEG400, or glycerol.

In a particular embodiment, the stable liquid eutectic system of the invention is a system of green tea catechins - choline salt - aprotic polar solvent in a molar ratio of 1.0-1.5:1:6-10. That is to say for 1 molar of the choline salt in said system, there are from 1 to 1.5 molar of green tea catechins and from 6 to 10 molar of aprotic solvent. Preferably, the molar ratio of green tea catechins - choline salt - aprotic polar solvent is 1.0-1.3:1:6-10.

In a more particular embodiment, the stable liquid eutectic system of the invention is a system of epigallocatechin gallate - choline chloride or choline tartrate - aprotic polar solvent in a molar ratio of 1.0-1.5:1:6-10. That is to say for 1 molar of the choline chloride in said system, there are from 1 to 1.5 molar of EGCG and from 6 to 10 molar of aprotic solvent. Preferably, the molar ratio of green tea catechins - choline chloride or choline tartrate - aprotic polar solvent is 1.0-1.3:1:6-10.

In a still more particular embodiment, the stable liquid eutectic system of the invention is a system of epigallocatechin gallate - choline chloride or choline tartrate - DMSO in a molar ratio of 1.0-1.5:1:6-10. That is to say for 1 molar of the choline chloride in said system, there are from 1 to 1.5 molar of EGCG and from 6 to 10 molar of DMSO. Preferably, the molar ratio of green tea catechins - choline chloride or choline tartrate - DMSO is 1.0-1.3:1:6-10.

In a still more particular embodiment, the stable liquid eutectic system of the invention is a system of epigallocatechin gallate - choline chloride - DMSO in a molar ratio of 1.2-1.3:1:6-7. That is to say for 1 molar of the choline chloride in said system, there are from 1.2 to 1.3 molar of EGCG and from 6 to 7 molar of DMSO. A particularly preferred system of the invention is a system of epigallocatechin gallate - choline chloride - DMSO in a molar ratio of 1.3:1:6.5.

In another particular embodiment, the stable liquid eutectic system of the invention is a system of green tea catechins - choline salt - protic polar solvent in a molar ratio of 0.25-1:1:15-50. That is to say for 1 molar of the choline salt in said system, there are from 0.25 to 1 molar of green tea catechins and from 15 to 50 molar of protic polar solvent.

In more particular embodiment, the stable liquid eutectic system of the invention is a system of epigallocatechin gallate - choline chloride - protic polar solvent in a molar ratio of 0.25-1:1:15-50. That is to say for 1 molar of the choline chloride in said system, there are from 0.25 to 1 molar of EGCG and from 15 to 50 molar of protic polar solvent.

In still more particular embodiment, the stable liquid eutectic system of the invention is a system of epigallocatechin gallate - choline chloride - water in a molar ratio of 0.25-1:1:15-50. That is to say for 1 molar of the choline chloride in said system, there are from 0.25 to 1 molar of EGCG and from 15 to 50 molar of water.

In a still more particular embodiment, the stable liquid eutectic system of the invention is a system of epigallocatechin gallate - choline chloride - water in a molar ratio of 0.65:1:25. That is to say for 1 molar of the choline chloride in said system, there are 0.65 molar of EGCG and 25 molar of water.

The method of preparation of a eutectic system of the present invention comprises :

- combining, under ambient temperature, an active ingredient, a choline salt, and an aprotic or protic polar solvent in a molar ratio of 0.25-3.5:1:5-75, preferably a molar ratio of 0.25-1.5:1:5-50, and
- mixing until complete liquefaction.

The ambient temperature is usually the acceptable temperature at the production floor of a pharmaceutical, dermo-cosmetic or food factory, e.g. controlled room temperature, such as from 15°C to 35°C, preferably from 15.0 to 29.0 °C, preferably from 18 °C to 28 °C, more preferably from 20.0 °C to 28.0 °C, still more preferably from 20.0 °C to 25.0 °C.

Another aspect of the present invention is to provide a pharmaceutical, dermo-cosmetic or nutraceutical composition, comprising a stable liquid eutectic system as defined before, and a pharmaceutically, dermo-cosmetically or nutraceutically acceptable carrier.

As used herein, the term "pharmaceutically acceptable carrier" is referred to any substance, within the scope of sound medical judgment, suitable for use in drug delivery which serves to improve the selectivity, effectiveness, and/or safety of the eutectic system administration, without causing any or a substantial adverse reaction.

The term "dermo-cosmetically acceptable carrier" is referred to any substance that is suitable for cutaneous application in general, and when applied they do not cause an unwanted toxicity, allergic response, redness, incompatibility, instability, and the like reactions. The term dermo-cosmetic essentially means cosmetic as referred to the skin.

The term "nutraceutical acceptable carrier" is referred to any substance that is suitable for oral administration to a human along with the eutectic system to improve the taste, mouthfeel, or colour of a nutraceutical composition, or to work as diluent, or to supply other nutritional value, without causing any or a substantial adverse reaction.

The carrier is "acceptable" in the sense of being not biologically or undesirable for human being.

According to the application and/or administration mode of said composition, a skilled person in the art can choose an appropriate carrier. For example, said pharmaceutically, dermo-cosmetically or nutraceutically acceptable carrier cans be aqueous vehicles, hydrophilic co-solvents, self-emulsifying drug-delivery systems (SEDDS), self-micro emulsifying drug-delivery systems (SMEDDS) or oil-free systems (micellar solutions), semi-solid materials, i.e. gels, emulsions, ointments, pastes and plasters.

In fact, besides high concentration in active ingredient, the liquid eutectic system of the present invention has another physicochemical advantage of being completely miscible with another aprotic or protic polar co-solvent, such as DMSO or water, which makes it possible to be formulated with aqueous vehicles, hydrophilic co-solvents, semi-solid materials, i.e. gels, emulsions, ointments, pastes and plasters or be loaded in self-emulsifying drug-delivery systems (SEDDS), self-micro emulsifying drug-delivery systems (SMEDDS) or oil-free systems (micellar solutions).

The term "miscible" means that the eutectic system of the present invention can be mixed with an aprotic or protic polar co-solvent to form a stable and homogenous mixture, without resulting in the formation of a separate phase.

This property facilitates the formulation of topically-applied or oral-administrated composition. By the way, addition of said eutectic system to the aforementioned vehicles does not change initial characteristics of said eutectic system.

A composition of the present invention can be obtained by directly mixing a eutectic system of the invention with hydrophilic co-solvents, self-emulsifying drug-delivery systems (SEDDS), self-micro emulsifying drug-delivery systems (SMEDDS) or oil-free systems (micellar solutions), or semi-solid materials.

Examples of semi-solid materials suitable to be used in pharmaceutical or dermo-cosmetic product formulations

can be gels, emulsions, ointments, pastes and plasters.

For example, gels can be single-phase hydrophilic gels containing 80 to 98 % water and comprising a network of hydrophilic macromolecules that trap aqueous, hydroalcoholic or glycolic solutions.

Examples of emulsion suitable to be mixed with the eutectic system of the invention can be multi-phase preparations composed of a lipophilic phase and an aqueous phase. Both lipophilic emulsions, which are water in oil (*W/O)* emulsions where the external phase is lipophilic, and hydrophilic emulsion, which are oil in water (*O/W)* emulsions where the external phase is aqueous, can be used. The *W/O* emulsions may contain emulsifying agents that stabilize *W/O* emulsions such as wool fat alcohols, sorbitan esters and monoglycerides. The *O/W* emulsions may contain emulsifying agents capable of stabilising *O/W* emulsions such as sodium or trolamine soaps, sulphated fatty alcohols, polysorbates and esters of polyoxyethylenated acids and fatty alcohols, possibly in combination with *W/O* emulsifying agents.

Hydrophilic emulsions are preferred.

Examples of ointments suitable to be mixed with the eutectic system of the invention consist of a single-phase base in which liquid or solid ingredients are solubilized or dispersed. They may be hydrophobic or hydrophilic, preferably hydrophilic ointments. These latter may be made of water-mixed excipients, such as a mixture of liquid and solid polyethylene glycol)s (macrogols).

Examples of pastes suitable to be mixed with the eutectic system of the invention are semi-solid preparations containing high proportions of solids dispersed in the base. Said pastes may be hydrophobic or hydrophilic, preferably hydrophilic such as water-based pastes. Plasters consist of an adhesive base spread in a single layer on a generally non-woven support. The device is covered with a protective film of polyethylene or polypropylene, which is removed before application without causing the preparation with it.

In a particular embodiment of the invention, the pharmaceutical, dermo-cosmetic or nutraceutical composition comprises:

- 0.1 % - 15.0 % wt, compared to total weight of composition, of a eutectic system as defined before, and further
- a pharmaceutically, dermo-cosmetically or nutraceutically acceptable polar aprotic co-solvent or polar protic co-solvent.

The term "pharmaceutically acceptable polar aprotic co-solvent or polar protic co-solvent" refers to any polar aprotic or protic co-solvent, within the scope of sound medical judgment, suitable for use in contact with the tissues of humans without undue toxicity, irritation, allergic response and the like.

The term "dermo-cosmetically acceptable polar aprotic co-solvent or polar protic co-solvent" refers to any polar aprotic or protic co-solvents, which are non-toxic for use in a dermo-cosmetic product.

The term "nutraceutically acceptable polar aprotic co-solvent or polar protic co-solvent" refers to any polar aprotic or protic co-solvents, which are suitable for use in contact with the tissues of humans without undue toxicity, irritation, allergic response and the like.

According to an embodiment of the invention, a non-exhaustive list examples of said protic polar co-solvent includes water, ethanol, propylene glycol, glycerol, PEG 400.

According to an embodiment of the invention, said aprotic polar co-solvent can be chosen from dimethyl sulfoxide, dimethylacetamide.

These solvents all have large dielectric constants (>20), but they do not participate in hydrogen bonding in the absence of O-H or N-H bonds.

In a particular embodiment of the invention, the pharmaceutical, dermo-cosmetic or nutraceutical composition comprises:

- 0.1 % - 15.0 % wt, particularly 0.5 % - 5 % wt, more particularly 1 % wt, compared to total weight of composition, of a eutectic system of EGCG as defined before, and further

a pharmaceutically, dermo-cosmetically or nutraceutically acceptable polar aprotic co-solvent or polar protic co-solvent.

The above composition may further be mixed with water or buffer solutions, hydrophilic co-solvents, self-emulsifying drug-delivery systems (SEDDS), self-micro emulsifying drug-delivery systems (SMEDDS) or oil-free systems (micellar solutions), or semi-solid materials, i.e. gels, emulsions, ointments and pastes.

According to an embodiment, the pharmaceutical, dermo-cosmetic or nutraceutical composition of the present invention is a stable aqueous solution of green tea catechins, in particular of EGCG. Said composition comprises a eutectic system of the invention and water or water saline buffer.

By the term "a stable aqueous solution of green tea catechins" is herein meant to an aqueous solution which conserves at least 90 % (w/w) of green tea catechins, compared to initial green tea catechins quantity in said solution after at least 6 months of storage at room temperature from 20 to 25 °C or at least 12 months of storage from 2 to 8 °C.

By the term "a stable aqueous solution of EGCG" is herein meant to an aqueous solution which conserves at

least 90 % (w/w) of EGCG and its trans-epimer, gallocatechin gallate (GCG), compared to initial EGCG quantity in said solution after at least 6 months of storage at room temperature from 20 to 25 °C or at least 12 months of storage from 2 to 8 °C.

Said water saline buffer can be a phosphate-buffered saline.

The pH of said stable aqueous solution of the invention is maintained in a pH range between 2.0 and 6.0, preferably between 3.0 and 5.0, in order to avoid an excessive risk of degradation of the EGCG due to the loss of a high pH proton (>5.0).

The pH of said buffer solutions may be adjusted by incorporation of suitable pharmaceutically, dermo-cosmetically or nutraceutically acceptable pH-modifying agents.

The term "pharmaceutically or nutraceutically acceptable pH-modifying agent" refers to any chemical agents which are, within the scope of sound medical judgment, suitable for use in contact with the tissues of humans without undue toxicity, irritation, allergic response and the like.

The term "dermo-cosmetically acceptable pH-modifying agent" refers to any chemical agents, which are non-toxic for use in a dermo-cosmetic product.

Generally, the pH-modifying agent can be an acidifying agent, especially an acid. Any suitable pharmaceutically acceptable acid may be used in the pharmaceutical solution of the present invention. Any suitable dermo-cosmetically acceptable acid may be used in the dermo-cosmetic solution of the present invention.

In an embodiment of the present invention, the pH-modifying agent is chosen from acetic acid, adipic acid, ammonium carbonate, ammonium hydroxide, ammonium phosphate, citric acid, diethanolamine, fumaric acid, hydrochloric acid, malic acid, nitric acid, proprionic acid, potassium acetate, potassium bicarbonate, potassium chloride, potassium citrate, potassium metaphosphate, potassium phosphate, sodium acetate, sodium bicarbonate, sodium carbonate, sodium citrate, sodium glycolate, sodium hydroxide, sodium lactate, sodium phosphate, sodium proprionate, succinic acid, sulfuric acid, tartaric acid, triethanolamine, or mixtures thereof.

The quantity of pH-modifying agent required is that which can confer to the solution the desired pH.

Sometimes, it may be necessary to incorporate a buffering agent to increase the pH of the solution to reach desired pH range. Any suitable pharmaceutically, dermo-cosmetically or nutraceutically acceptable buffering agent may be respectively used in a pharmaceutical, dermo-cosmetic or nutraceutical aqueous solutions of the present invention.

Sometimes, the stable aqueous solutions of the present invention may need to comprise a chelating agent to capture any trace metal ions potentially present in the solution and preventing green tea catechins deprotonation which can be catalysed in the presence of metal ions even at trace concentration.

As used herein, the term "chelating agent" refers to a chemical compound that reacts with metal ions to form stable, water-soluble metal complexes.

Examples of suitable chelating agent for a solution of the present invention are citric acid, calcium disodium edetate, disodium edetate, fumaric acid, malic acid, maltol and pentetic acid.

According to an embodiment of the present invention, a chelating agent and a pH-modifying agent can be a same compound. For example, citric acid is on the same time a chelating agent and a pH-modifying agent.

According to a preferred embodiment of the present invention, the stable aqueous solution of the present invention further comprises at least one antioxidant agent suitable for the aqueous systems and compatible with the pH range mentioned above.

Examples of suitable antioxidant agent are ascorbic acid, erythorbic acid, monothioglycerol, sodium metabisulfite, sodium bisulfite, or reducing sugar.

In a preferred embodiment, when the stable aqueous solution of the present invention comprises an antioxidant chosen from ascorbic acid (vitamin C), erythorbic acid, monothioglycerol, sodium metabisulfite, or sodium bisulfite, the concentration of said antioxidant is in the range of from 0.01 mg/mL to 0.5 mg/mL.

In another particular embodiment of the invention, the pharmaceutical, dermo-cosmetic or nutraceutical composition comprises:

- 0.1 % - 15.0 % wt, compared to total weight of composition, of a eutectic system as defined before, and further
- 0.1-1.0 mmol/L of at least one chelating agent,
- water or water saline buffer,
- optionally at least one another antioxidant,

wherein said solution is at a pH in a range of 2.0-6.0, preferably 3.0-5.0.

In a more preferred embodiment, the pharmaceutical, dermo-cosmetic or nutraceutical composition, in particular the stable aqueous solution of the invention, further comprises vitamin C.

Another aspect of the present invention concerns a solid composition resulting from any water removal process, especially lyophilisation or spray drying, of the aqueous solution described before.

Said solid composition can be of amorphous, crystalline, hydrate or anhydrous polymorph forms.

A pharmaceutical, dermo-cosmetic or nutraceutical aqueous solution described before can be re-established from said solid composition by adding suitable volume of water or of the aforementioned buffer solutions.

The present invention also provides a pharmaceutical or nutraceutical composition, resulting from the aforementioned solid composition, dispersed within solid excipients. Said solid excipients can be any suitable solid excipient for drug formulation well known by a skilled person, such as that described in the Handbook of Excipients (ISBN 978 0 85369 792 3 (UK); ISBN 978 1 58212 135 2 (USA)).

The present invention also provides a semi-solid or pasty topical applied pharmaceutical or dermo-cosmetic composition comprising the aforementioned solid composition, resulting from the dispersion of the aforementioned solid composition within different pasty or semi-solid bases, such as gels, emulsions, ointments and pastes.

In a particular embodiment, a pharmaceutical composition of the invention as described above is formulated in a self-emulsifying drug-delivery system, a self-micro emulsifying drug-delivery system or an oil-free system.

In specific embodiments, the present invention concerns a pharmaceutical or a dermo-cosmetic composition, said composition being formulated to be a semi-solid or pasty topical composition.

In a more specific embodiment, the present invention concerns a pharmaceutical or dermo-cosmetic semi-solid or pasty topical composition, comprising or consisting of:

- 0.5 % to 10.0 % wt, particularly 0.5 % wt to 5.0 % wt, more particularly 1 % wt, compared to total weight of composition, of the aforementioned solid composition,
- a pharmaceutically or dermo-cosmetically acceptable pasty or semi-solid bases.

The present invention provides also a unit dose of the stable liquid eutectic system as described before, or a unit dose of the liquid composition comprising said eutectic system as described before, or a unit dose of the stable aqueous solution comprising said eutectic system as described before, or a unit dose of the solid composition resulting from any water removal process of said aqueous solution as described before, or a unit dose of the semi-solid or pasty topical composition as described before.

The present invention provides a pharmaceutical composition comprising above described eutectic system, or the aforementioned liquid composition comprising said eutectic system, or the aforementioned solid composition, for use as a medicament in the treatment of cancers, cardiovascular disorder, diabetes, neurodegenerative diseases, such as Parkinson's disease and Alzheimer's disease, or dermatological diseases.

Administration can be carried out by oral administration routes.

The present invention concerns also the above-mentioned semi-solid or pasty topical pharmaceutical composition for its use as medicament in the treatment of dermatological diseases.

Administration can be carried out by topical routes.

The present invention also provides a dermo-cosmetic product comprising the aforementioned stable aqueous solution, or the aforementioned solid composition resulting from any water removal process of said stable aqueous solution, or a unit dose thereof.

Said dermo-cosmetic product can be an aqueous solution or a semi-solid or pasty topical composition.

Said dermo-cosmetic product can be a skin care product, such as sun creams, day creams, night creams, after-sun products, makeup's, lipsticks, eye cosmetics, shampoos, showers.

The present invention also provides an oral care comprising the aforementioned stable aqueous solution, or the aforementioned solid composition resulting from any water removal process of said stable aqueous solution, or a unit dose thereof.

Said oral care can be formulated as a semi-solid or pasty topical composition.

Examples of oral care products according to the invention which can be cited are toothpastes, gingival and oral gels, solutions and sprays.

The present invention also provides a food or a drink comprising a stable liquid eutectic system as described before, or a nutraceutical composition as described before, said food or drink comprises at least 10 mg of green tea catechins, in particular 50 mg of epigallocatechin gallate, from the eutectic system as described before.

The present invention also provides a food or a drink comprising a stable liquid eutectic system as described before, or a nutraceutical composition as described before, said food or drink comprises at least 10 mg of green tea catechins, in particular 50 mg of epigallocatechin gallate, being present in the eutectic system as described before.

Said food or drink can be prepared by adding a suitable quantity of eutectic system of the present invention, in particular a suitable quantity of EGCG-choline salt-water eutectic system, more in particular at least 30 mg of EGCG -choline chloride-water eutectic system to said food or drink.

Said eutectic system may be added in the last step of fabrication process, just before food or drink packaging.

In a particular embodiment, the food or the drink according to the invention is coffee capsule comprising at least 10 mg of epigallocatechin gallate per gram of coffee, being present in the eutectic system as described before.

Said coffee capsule can be obtained by directly adding a suitable quantity of EGCG-choline salt-water eutectic

system, in particular 30 mg of EGCG -choline chloride-water eutectic system per gram of coffee.

Preferably, the eutectic system may be added just before encapsulation of coffee.

In another particular embodiment, the food or the drink according to the invention is tea capsule comprising at least 10 mg of epigallocatechin gallate per gram of tea (white, green, oolong, black), from the eutectic system as described before.

Said tea capsule can be obtained by directly adding 30 mg of EGCG - choline chloride-water eutectic system per gram of tea.

Preferably, the eutectic system may be added just before encapsulation of tea.

In a particular embodiment, the food or the drink according to the invention is any type of ready-to-use powder concentrates (milk, chocolate, coca cola, *ricoré* or any other coffee surrogate, cappuccino,...) to be dissolved in water or other beverages comprising at least 10 mg of epigallocatechin gallate per gram, from the eutectic system as described before.

Said tea capsule can be obtained by directly adding 10 mg of EGCG - choline chloride-water eutectic system per gram of concentrate.

In another aspect, a method for preparing an aqueous solution containing an active ingredient chosen from catechins, anthocyanins, and procyanidins, particularly green tea catechins, more particularly EGCG is described.

Said method comprises following steps:

(i) preparing a stable liquid eutectic system comprising or consisting of :

- an active ingredient chosen from catechins, anthocyanins, and procyanidins,
- a choline salt,
- a polar protic solvent or aprotic polar solvent which is pharmaceutically, dermo-cosmetically or nutraceutically acceptable,

wherein the molar ratio between the active ingredient, the choline salt, and the solvent is 0.25-3.5:1:5-75, preferable 0.25-1.5:1:5-50, and
wherein the concentration of the active ingredient is at least 100 mg/mL,

(ii) preparing an aqueous solution containing:

- drinking water, injectable water, purified water, demineralized water or spring waters,
- optionally, 0.1-1.0 mmol/L of at least one chelating agent,
- optionally, a pharmaceutically acceptable pH-modifying agent to adjust the pH of solution in a range of 3.0 to 5.0,
- optionally, at least one antioxidant agent suitable for the aqueous systems and compatible with acidic pH,

(iii) dissolving 0.5 % to 15.0 % wt, particularly 0.5 % to 10.0 % wt, more particularly 7.0 % wt, of total weight of the stable liquid eutectic system in the aqueous solution prepared in step (ii) to obtain a stable aqueous solution containing the active ingredient.

Described is also a method for improving green tea catechins, in particular EGCG, bioavailability.

Said method comprises following steps:

(i) preparing a stable aqueous solution containing green tea catechins according to the above-mentioned method,
(ii) optionally, mixing said stable aqueous solution with hydrophilic co-solvents, self-emulsifying drug-delivery systems (SEDDS), self-micro emulsifying drug-delivery systems (SMEDDS) or oil-free systems (micellar solutions) to obtain a mixture,
(iii) administrating said stable aqueous solution or said mixture to a patient in need of treatment by green tea catechins, in particular EGCG.

Described is also a method for improving green tea catechins, in particular EGCG, bioavailability.

Said method comprises following steps :

(i) preparing a stable aqueous solution containing green tea catechins according to the above-mentioned method,
(ii) optionally, removing water by using any water removal process, especially lyophilisation or spray drying from said solution to afford a solid composition,
(iii) administrating said solution or solid composition by topical route to a patient in need of treatment by green tea catechins, in particular EGCG.

Described is also a method for improving green tea catechins, in particular EGCG, skin permeation.

Said method comprises following steps :

(i) preparing a stable aqueous solution containing green tea catechins according to the above-mentioned method,
(ii) optionally, mixing said aqueous solution with hydrophilic co-solvents, self-emulsifying drug-delivery systems (SEDDS), self-micro emulsifying drug-delivery systems (SMEDDS) or oil-free systems (micellar solutions), semi-solid materials, i.e. gels, emulsions, ointments, pastes and plasters to obtain a mixture,
(iii) applying said solutions or mixtures to a patient in need of treatment by green tea catechins, in particular EGCG.

The present invention is illustrated in more detail by the following figures and examples.

**Figures**

Figure 1 compares HPLC profiles of an aqueous EGCG composition INV1 (formulation showed in table 2 below) exposed to alkali conditions at initial time (1A) and after a 6-day exposure (1B) and that of reference EGCG water suspension also after a 6-day exposure in the same stress conditions (1C).
Figure 2 compares HPLC profiles of the EGCG composition INV1 exposed to 70 °C at initial time (2A) and after a 6-day exposure (2B) and that of reference EGCG water suspension also after a 6-day exposure in the same stress conditions (2C).
Figure 3 compares HPLC profiles of the EGCG composition INV1 exposed to photolysis conditions at initial time (3A) and after a 6-day exposure (3B) and that of reference EGCG water suspension also after a 6-day exposure in the same stress conditions (3C).
Figure 4 shows ATR-IR spectra of eutectic system formed by EGCG, choline and DMSO (4A) and that of EGCG powder (4B).
Figure 5 compares *in vitro* cumulative percentage of EGCG permeation over time of a EGCG cream formulation INV2 (represented in the figure by diamond) and that of control formulation (represented in the figure by square) through Franz diffusion cells membrane.
Figure 6 shows the evolution over time of plasma EGCG concentrations in primates after oral administration of a control formula (capsules dosed to administer 27 mg EGCG per kg) and the aqueous EGCG composition INV1 (27 mg EGCG per mL).

## EXAMPLES

EXAMPLE 1: EGCG SOLUBILITY AND STABILITY

### 1. Materials and methods

### 1.1 Tested EGCG composition

An EGCG aqueous composition according to the present invention comprising 26.7 mg/mL of EGCG is prepared by using an EGCG-choline chloride-DMSO eutectic system of the present invention. This composition is named composition INV1 in the description.

The reference standard suspension of EGCG used for the stress stability testing, or long-term and accelerated stability studies is a water suspension containing uniquely EGCG at a concentration of 26.6 mg/mL. The standard suspension is extemporaneously prepared by dispersing an appropriate amount of an isolated EGCG extract (> 97 % w/w of the extract dry) in distilled water to afford the said concentration.

#### 1.1.1 EGCG-choline chloride-DMSO liquid eutectic system formula

**Formulation**

A EGCG-choline chloride-DMSO liquid eutectic system according to the invention is prepared according to the formulation given in Table 1 below.

**Table 1: example of the formula of a liquid eutectic system EGCG-choline chloride-DMSO**

| Ingredients | For 1mL volume | For 1 l volume |
|---|---|---|
| EGCG | 595 mg | 0.595 kg |
| DMSO | 507 mg | 0.507 kg |
| Choline chloride | 140 mg | 0.140 kg |

EGCG used for preparing said system is an isolated EGCG extract (> 97 % w/w of the extract dry). Prior to its use, the active substance was thoroughly controlled to ensure its quality comply with Monograph 2668 (Pharmeuropa June 2017).

**Protocol of preparation**

An example of manufacturing process of said liquid eutectic system is given below.

**Stage 1: preparation of the EGCG-choline chloride-DMSO liquid eutectic system**

Step1: a suitable quantity of DMSO according to Table 1 is poured into a 1-liter Pyrex glass vessel.
Step2: a suitable quantity of choline chloride as mentioned in table 1 is added to the solvent and the mixture is stirring until choline chloride solubilizes.
Step3: an appropriate amount of EGCG according to table 1 is added to the mixture from step 2 while maintaining stirring until any particles or lumps are completely dissolved.

**Stage 2: Filling into multiple-dose bottle**

The solution is filled into 15 mL single-dose amber glass bottles. Filled bottles are closed with caps with a polyethylene seal.

**1.1.2 Aqueous EGCG composition formula at 26.7 mg/mL**

**Formulation**

The liquid eutectic system obtained before is used for preparing the aqueous EGCG composition named INV1 comprising 26.7 mg/mL of EGCG. The formulation of this composition is given in Table 2 below.

**Table 2: example EGCG 26.7 mg/mL solution formula**

| | Quantities | |
|---|---|---|
| | 26.67 mg/mL EGCG aqueous solution | |
| | Unit formula (15 mL) | Formula for 20 L of solution |
| EGCG | 400 mg | 0.533 kg |
| DMSO | 340 mg | 0.453 kg |
| Choline chloride | 95 mg | 0.127 kg |
| Citric acid | 4.5 mg | 0.006 kg |
| Purified water | Qs 15 mL | Qs 20 L |

EGCG used for preparing said solution is an isolated EGCG extract (> 97 % w/w of the extract dry). Prior to its use, the active substance was thoroughly controlled to ensure its quality comply with Monograph 2668 (Pharmeuropa June 2017).

**Protocol of preparation**

An example of manufacturing process of said aqueous solution is given below.

**Stage 1: preparation of the EGCG-choline chloride-DMSO liquid eutectic system**

Step1: a suitable quantity of DMSO according to Table 2 is poured into a 20-liter Pyrex glass vessel.

Step2: a suitable quantity of choline chloride as mentioned in table 2 is added to the solvent and the mixture is stirring until choline chloride solubilizes.

Step3: an appropriate amount of EGCG according to Table 2 is added to the mixture from step 2 while maintaining stirring until any particles or lumps are completely dissolved.

**Stage 2: Aqueous solution preparation**

Step1: purified water is added to reach 2/3 of the final volume while maintaining agitation until a clear and homogeneous aqueous solution is obtained.

Step2: an appropriate amount of citric acid according to Table 2 is added to the mixture from step 1. The mixture is stirred until the citric acid is completely dissolved.

**Stage 3: Filtration**

Filtration is carried out by a 1μm Millipore® filtration cartridge. The filtered solution is stocked in a 20 liter-stainless steel tank with nitrogen inlet for inerting the solution and the headspace of the container.

**Stage 4: Filling into multiple-dose bottle**

The solution is filled into 15 mL single-dose amber glass bottles. Filled bottles are closed with caps with a polyethylene seal.

**1.2 Stress stability testing:**

1 mL of the reference standard suspension in water and 1 mL of the aqueous composition INV1 were exposed to alkali, thermal and photolysis stress conditions up to 6 days. These conditions, described in Table 3, were chosen to assess the stability of EGCG because they are known to cause its degradation. At the end of exposure, the solutions were diluted at 1/100 in distilled water and analyzed by high performance liquid chromatography (HPLC). The results are displayed in Figures 1-3.

**Table 3 - Stress conditions to assess EGCG stability**

| Stress conditions | Descriptions | Exposure duration |
|---|---|---|
| Basic condition | Dilution of samples to be tested at 1/2 in a 0.1 mol/L NaOH solution. | Up to 6 days |
| Photolysis conditions | The source used produces an output similar to the D65/ID65 emission standard. These irradiation conditions provide an overall illumination of not less than 1.2 million lux hours. | Up to 6 days |
| Thermolysis conditions | 70 °C | Up to 6 days |

**1.3 Long-term and accelerated stability testing**

Storage conditions and sampling protocols

The tested batch is the aqueous composition INV1 prepared according to the formulation aforementioned in Table 2.

Reference standard solution of EGCG is a water solution containing uniquely EGCG at a concentration of 0.26 mg/mL.

The tested batch has been stored in accordance with ICH (The International Council for Harmonisation of Technical Requirements for Pharmaceuticals for Human Use) guideline Q1A(R2) (https://www.ich.org/products/guidelines/quality/quality-single/article/stability-testing-of-new-drug-substances-and-products.html). Details of the sampling time points for each condition are presented in Table 4. It is diluted 1/100 in distilled water prior to HPLC analysis.

**Table 4 - Sampling and storage protocol for primary batch studies**

| Time (months) | Storage conditions / container | |
|---|---|---|
| | 2-8°C Pack | 25°C/60% RH Pack |
| 1 | + | + |
| 2 | + | + |
| 3 | + | + |
| 4 | + | + |
| 5 | + | + |
| 6 | + | + |
| 9 | + | + |
| 12 | + | + |
| 18 | + | + |
| Pack: 15 mL-single-dose amber glass bottle (Type III) with a polyethylene cap with polyethylene seal. | | |

Stability tests and acceptance limits

Monitoring appropriate chemical, physical and microbial characteristics during the stability studies and at each time point has allowed assessing the stability of EGCG aqueous solution.

The chemical stability of EGCG was evaluated by HPLC, validated assay method as a stability indicating method. Said procedure is suitable for the EGCG investigational product assay using an external standard. The assay of EGCG is achieved by comparing the response of a sample solution with the response of EGCG reference standard solution prepared at a similar nominal concentration and analysed in the same way. The sample and reference standard solutions are analysed by gradient reversed phase HPLC and UV detection using a suitable column and chromatography conditions.

HPLC assay uses a liquid chromatograph fitted with a UV detector (variable wavelength or diode array detector) and equipped with column of Interchim® VKR5 C18 (250 × 4.6.0 mm, 5 μm particle size), or equivalent.

Acetonitrile, acetic acid and pure water are used for prepare mobile phase A (5/95/0.07 v/v/v Acetonitrile/Water/Acetic acid) and mobile phase B (50/50/0.05 v/v/v Acetonitrile/Water/Acetic acid).

Chromatography Procedure

Analyse using suitable chromatographic conditions, for example, those given in Table 5.

**Table 5 - Chromatographic conditions for the assay of EGCG**

<table>
<tr><th>Parameter</th><th>Setting</th></tr>
<tr><td>Mobile phase A</td><td>Prepared as described before</td></tr>
<tr><td>Mobile phase B</td><td>Prepared as described before</td></tr>
<tr><td>Flow rate</td><td>1 mL/min (or as appropriate)</td></tr>
<tr><td>Oven temperature</td><td>32°C</td></tr>
<tr><td>Detection wavelength</td><td>275 nm</td></tr>
<tr><td>Injection volume</td><td>20 µL</td></tr>
<tr><td>Gradient programme</td><td>The gradient program was set as follows:
<table>
<tr><th>Temps (min)</th><th>A (%)</th><th>B (%)</th></tr>
<tr><td>0</td><td>90</td><td>10</td></tr>
<tr><td>10</td><td>80</td><td>20</td></tr>
<tr><td>16</td><td>60</td><td>40</td></tr>
<tr><td>20</td><td>50</td><td>50</td></tr>
<tr><td>25</td><td>50</td><td>50</td></tr>
<tr><td>27</td><td>60</td><td>40</td></tr>
<tr><td>30</td><td>90</td><td>10</td></tr>
<tr><td>33</td><td>90</td><td>10</td></tr>
</table></td></tr>
<tr><td>Approximate equilibration time</td><td>45 min</td></tr>
<tr><td>Approximate retention time of EGCG</td><td>≈ 15 min</td></tr>
</table>

System suitability criteria

All system suitability criteria are calculated according to the current European Pharmacopoeia "Chapter 2.2.46". Specific parameters of the procedure may be modified within pharmacopoeial and validated limits, if necessary, to achieve system suitability.

System suitability criteria should be met are displayed in Table 6.

**Table 6 - System suitability criteria**

| System suitability test | Acceptance criteria |
|---|---|
| Tailing factor | Confirm that the tailing factor of the EGCG peak from a reference standard solution injection is not greater than 1.5. |
| System repeatability | Confirm that the relative standard deviation for 3 injections from a reference standard solution is not greater than 2.0% |

Calculation

The percentage of quantity of remained EGCG in diluted sample solution compared to original quantity in that sample is calculated according to following formula

$$\text{EGCG (\% of original quantity)} = \frac{\text{Wstd} \times \text{Pstd} \times \text{Asam} \times \text{D}}{\text{Astd} \times \text{Vstd} \times 26.67} \times 100$$

Wherein:

$A_{std}$ = Peak area due to EGCG in reference standard solution
$A_{sam}$ = Peak area due to EGCG in diluted aqueous composition INV1
D = Dilution factor
$W_{std}$ = Weight of EGCG taken for the reference standard solution (mg)
$P_{std}$ = Purity of reference standard solution (% w/w)
$V_{std}$ = Volume of reference standard solution (mL)

**1.5 ATR-IR spectres image**

ATR-IR spectra image of eutectic system EGCG/choline chloride/DMSO is obtained by FTIR-spectroscopy. The experiments were performed on a Perkin-Elmer Spectrum BX FT-IR system based on diffuse reflectance sampling accessories with FT-IR Spectrum v2.00 software. The spectra of the stressed samples were recorded at room temperature in the wavenumber range of 400-4000 $cm^{-1}$ using ATR cell.

**1.6 Solubility**

The Crystal16 equipment of Technobis BV has been used to determine the solubility of said EGCG eutectic system in ultrapure water. In the Crystal16, cloud points and clear points of sixteen 1 mL solution aliquots can be measured in parallel and automatically, based on turbidity. The temperature at the point the suspension becomes a clear solution upon heating (at 0.5 °C per minute) is generally taken as the saturation temperature of the measured sample, of which the composition is established beforehand. The maximum error (overshoot) in the saturation temperature measured in this way, because the conditions are not strictly at equilibrium, is in the order of <0.5 °C.

**2. Results**

**2.1 Solubility of EGCG according to the invention**

The EGCG liquid eutectic system were extremely soluble and no temperature ramp has been used. Samples were kept at 25°C with 600 rpm stirring. Water solutions were prepared with said liquid eutectic system of the following concentrations: 60, 75, 100.8, 120, 150, 199.9, 310.7 and 407.3 mg/mL. According to Table 1, the effective concentrations of EGCG in these water solutions are respectively the follows: 28.8, 36, 48.4, 57.6, 72, 95.9, 149.1, 195.5 mg/mL.

All solutions became completely clear after several minutes of stirring, indicating that all concentrations were basically soluble. Compared to the intrinsic solubility of EGCG in water at room temperature, i.e. 4.6 mg/mL, the EGCG eutectic system of the present invention allows EGCG to gain solubility by a factor of at least 32.

**2.2 Stability of EGCG solution according to the invention**

Stability tests as described in section 1.2 are carried out for the EGCG aqueous composition INV1 and an EGCG-based suspension alone as reference. After six days of exposure to the various stress conditions mentioned in Table 3, the samples were subjected to HPLC analysis. The reference EGCG-based suspension alone contains practically no more EGCG in solution when exposed to alkali conditions (Figure 1C), losses 49.5 % EGCG when subjected to high temperature (Figure 2C) and losses 22.2 % under photolysis conditions (Figure 3C), whereas the chromatographic profiles of the product resulting from the heat treatment, alkali treatment or photolysis treatment of the EGCG aqueous composition INV1 (Figures 1B, 2B, 3B) is identical to that of the starting solution (Figures 1A, 2A, 3A), namely that it represents the same as the peak due to EGCG and that of another polyphenol of the green tea initially present.

The results of long-term stability performed on the EGCG aqueous composition INV1 are given in Table 7 below.

**Table 7: long-term stability test**

| Time (months) | Relative content of EGCG with respect to the initial time (%) for each storage conditions | |
|---|---|---|
| | 2-8°C | 25°C/60% RH |
| 1 | > 99 % | > 99 % |
| 3 | > 99 % | > 99 % |
| 6 | > 99 % | > 99 % |
| 9 | Analysis planned | Analysis planned |
| 12 | Analysis planned | Analysis planned |
| 18 | Analysis planned | Analysis planned |

Long-term stability studies, up to now, show that the EGCG levels present in EGCG aqueous composition INV1 (26.7 mg/mL) remained unchanged at both storage conditions, i.e. 2-8°C and 25°C/ 60% HR.

These results suggest that the formulations developed were able to protect EGCG from auto-oxidation, hydrolysis and photolysis.

### 2.3 Structure of eutectic system

ATR-IR spectres image shows the presence of broad absorption in the O-H stretch IR regions and the absence of O-H free stretch peaks (Figure 4A), that suggests that with OH functions DMSO and choline chloride as acceptors, EGCG would form eutectic system with DMSO and choline chloride by strong hydrogen bonds OH-O.

H-bonding may have caused vibrational modes to vibrate at lower frequencies and/or relative intensities than before, as long as the atoms involved in the vibrational modes are actively participating in H-bonding.

## EXAMPLE 2: *IN VITRO* PERMEABILITY STUDY

In this study, Franz diffusion cells were used to compare the membrane passage of EGCG from a cream control formulation and a cream formulation of the present invention.

### 1. Materials and methods

The tested EGCG topical formulation named INV2 is obtained by mixing the eutectic liquid EGCG eutectic system presented in Table 1 with a commercially available moisturizing serum chosen at random on the market. The control EGCG-containing cream was made from EGCG powder directly mixed with the same serum. Both formulations contain an equivalent amount of 1 % *w*/*w* EGCG.

***Permeability assessment using Franz diffusion cells***

Drug release and skin permeation were determined using Franz diffusion cells.

The experiments were conducted in three independent vertical Franz cells with a nominal volume of the acceptor compartment of 125 mL and a diffusion area of 1.767 $cm^2$. For the donor compartment, an amount of 1 mL of each formulation (1 % w/w EGCG) was initially set. The experiments were conducted in triplicate, carried out at 32°C and 400 rpm for 24 h. Samples were evaluated at different time points, and data analysis was made by comparing the releasing efficiency (PE) values.

The assays were performed in an aqueous medium as acceptor phase mimicking physiological conditions corresponding to 0.15 mol/L PBS® buffer solution at pH 7.4. A regenerated cellulose membrane with molecular weight cutoff at 12 kDa was used.

Releasing efficiency was defined in terms of the mass flux (J), which describes the change of drug permeation with respect to time in aqueous systems. In this study, the mass flux (mol $cm^{-2}$ $h^{-1}$) was determined using the AUC of the permeation profile recorded at a specific time interval and is related to the rectangular area (R) described by 100 % of the permeation process at the same time interval (24 h).

Mass flux can be calculated from:

$$Flux\ (J) = \frac{\int_0^t y\, dt}{y_{100}t} \times 100\%$$

where $y_{100}$ is the AUC value assumed with a permeation of 100 % in a time interval t, and y represents the AUC value of the permeated drug during the same time interval.

A 0.5 mL aliquot was withdrawn periodically at pre-set time from the abovementioned receiver cell, which was thereafter 10 times diluted with *PBS* buffer solution and filtered through 0.45 μm filter. EGCG content was determined by HPLC. The diffusion fluid of the same volume was pre-warmed at 32°C. The volume of withdrawn samples was replaced by pre-warmed diffusion fluid into the diffusion cell to keep the volume constant so that sink condition could be maintained.

The cumulative amounts of drug in the receptor during 24 h were plotted after administration of various tested and control formulations as shown in Fig. 5.

***HPLC Analysis***

The diluted samples were analysed by HPLC, validated assay method as a stability indicating method described above in Example 1. Said procedure is suitable for EGCG assay using an external standard. The assay of EGCG is achieved by comparing the response of a sample solution with the response of EGCG reference standard solution prepared at a similar nominal concentration and analysed in the same way. The sample and reference standard solutions are analysed by gradient reversed phase HPLC and UV detection using a suitable column and chromatography conditions.

**2. Results**

Permeability study of the two formulations of EGCG (formulation INV2 versus control EGCG containing cream) was carried out using cellulosic membrane as diffusional membrane and simulated skin medium as diffusional fluid at different time points up to 24 h.

The cumulative amounts of drug in the receptor during 24 h were plotted after administration of various formulations as shown in Figure 5. EGCG flux across the skin was significantly lower than that released from the formulation of the present invention (Table 8). EGCG of the present invention significantly increased *in vitro* drug permeation compared to the control.

These results clearly show that the formulation INV2 of present invention significantly improves the skin permeation of EGCG, paving the way for improved clinical efficacy of a EGCG containing dermo-cosmetic product or a pharmaceutical dermal product.

**Table 8: AUC values for mass flux and lag time obtained from the EGCG permeation profiles of the tested and control formulations using cellulose membrane at 32°C.**

| Type of 1 % w/w EGCG in a marketed moisturizing serum | Flux (μg $cm^{-2}$ $h^{-1}$) | Lag-time (h) |
|---|---|---|
| EGCG cream composition INV2 | 1.29 | 1.72 |
| EGCG powder in serum (control) | 0.81 | 1.65 |

**EXAMPLE 3: PLASMA CONCENTRATIONS OF EGCG IN PRIMATES**

This test was performed to compare plasma concentrations and total area under the curver ($AUC_{total}$) obtained after oral administration in *Cynomolgus* monkeys of the EGCG aqueous composition INV1 of the present invention with that of EGCG containing capsules as control formulation.

**1. In-life phase**

The content of EGCG in tested capsules are adapted according to the weight of monkey to ensure an admiration of 26.67 mg EGCG / kg of monkey weight.

The treatment patterns of two tested formulations are listed below:

| Name | Form | Content | Other details |
|---|---|---|---|
| EGCG capsules | Capsules | Monkey 1: 145.8 mg of EGCG per capsule<br>Monkey 2: 116.1 mg of EGCG per capsule<br>Monkey 3: 108.0 mg of EGCG per capsule | Content pre-adjusted to the weight of the monkey.<br>2 capsules for each monkey were provided. However, only one capsule is to be administered to each monkey. Please rely on labeling. |
| EGCG aqueous composition INV1 | Solution | 26.7 mg/mL | The volume administered is to be adjusted according to the weight of the monkey.<br>Shake well before sampling. |

Formulations were stored at room temperature and well shaken before sampling.

**2. Animals and husbandry**

The conditions of monkey breeding are summarized below.

| | | | | |
|---|---|---|---|---|
| **Species:** | Monkey | | **Strain/Breed:** | Cynomolgus |
| **Number:** | 3 | **Weight:** | About 3-6 kg | **Age:** adult |
| **Status:** | Non naive | | **Supplier:** | Avogadro LS colony |
| **Acclimatization:** | At least 7 days | | **Housing:** | Per group of 3 and individual condition during the blood sampling collection time until 3h post dosing |
| **Daily light cycle:** | 12h day/ 12h night | | **Temperature range:** | 20 - 24 °C |
| **Feeding conditions:** | Ration: | Regular | Overnight fasting: | No |
| **Fate of animals:** | Return to stock | | **Necropsy:** | On unexpected dead animals only |

**3. Study design and dosing details**

EGCG aqueous composition INV1 was orally administered by gavage using a gastro-oesophageal tube or a nasogastric tube and an appropriate syringe. After administration, the tube was rinsed with 5 mL of water and flushed with 5 mL of air to ensure that the entire dose will be delivered.

Capsule of EGCG was administered using a specific device to place the item on the back of the tongue.

The wash out period is at least 7 days.

| Period | Formulation administered | Formulation concentration | Target dose | Quantity to administer per animal |
|---|---|---|---|---|
| 1 | EGCG capsule | 145.8 mg/capsule<br>116.1 mg/capsule<br>108.0 mg/capsule | 27 mg/kg | 1 capsule |
| 2 | EGCG Eutectic solution | 26.7 mg/mL | 27 mg/kg | Monkey 1: 5.5 mL<br>Monkey 2: 4.3 mL<br>Monkey 3: 4.1 mL |

### 4. Blood sampling and processing

| | | | |
|---|---|---|---|
| **Blood collection:** | Volume 0.5 mL | Tube: LH | Total number of 108 samples: |
| | Vein: Cephalic or femoral | | |
| | Sampling times: | 30 min, 1 h, 1.5 h, 2 h, 2.5 h, 3 h, 3.5 h, 4 h, 5 h, 7 h, 12h, 24h | |
| **Plasma preparation:** | Centrifugation: | 2500 g / 10 min / 5°C | Number of 2 (A & B) aliquots: |
| | Distribution: | Eppendorf tubes | |
| | Aliquot volume: | A: 100 μL | B: remaining plasma (at least 100 μL) |
| | Storage until shipping: | ca. -80°C | |

### 5. Analytical test

The analytical test was based on the method described by de Lourdes et al (J. Agric. Food Chem. 2007, 55, 8857-8863).

The molecular and daughter ions were selected for each molecule after direct infusion into the MS-MS system. The analytical method consisted of a precipitation of the proteins by addition of appropriate solvent followed by a LC-MS/MS analysis. According to the expected sensitivity, at least 8 calibration standards were used for the preparation of the calibration curve in plasma. The corresponding correlation coefficient were calculated and had to be higher than 0.75 to continue with the *in vivo* test.

The calibration range tested will be 4 to 5000 ng/mL of EGCG in plasma. The difference between the mean concentration observed and the nominal concentration was used to estimate the deviation of the method.

### 6. Results

EGCG plasma concentration and $AUC_{total}$ results are summarized in the table below (Table 9).

Figure 6 illustrates the evolution of plasma EGCG concentrations over time.

As these results show, EGCG plasma concentration resulting from the administration of the composition INV1 is, at equal doses administered, much higher than that resulting from the administration of capsule containing 100 % of the EGCG powder. Indeed, the total AUC ratio per dose of administration of the composition INV1 is 6 times higher than that of EGCG-containing administration.

Table 9: Plasma concentration and PK parameters

| **Treatment** | **$T_{max(h)}$** | **$C_{max}$ (ng/mL)** | **AUC0-24 (ng/mL.h)** | **Kel(1/h)** | **T1/2(h)** | **AUCt (ng/mL.h)** | **AUCinf (ng/mL.h)** | **%$AUC_{extra}$** | **$r^2$** |
|---|---|---|---|---|---|---|---|---|---|
| EGCG capsule (n=3) | 2.33 | 510.56 | 1181 | 0.12 | 6.05 | 1155 | 1219 | 5.42 | 0.94 |
| EGCG formulation (n=3) | 1 | 2996.1 | 6781 | 0.11 | 6.52 | 6781 | 6981 | 3.04 | 0.99 |
| ratio | 0.4 | 5.9 | 5.7 | 0.9 | 1.1 | 5.9 | 5.7 | 0.6 | 1.1 |

## Claims

1. A stable liquid eutectic system comprising :

   - an active ingredient chosen from catechins,
   - a choline salt,
   - a polar protic solvent or aprotic polar solvent which is pharmaceutically, dermo-cosmetically or nutraceutically acceptable,

   wherein the molar ratio between the active ingredient, the choline salt, and the solvent is 0.25-3.5:1:5-75. wherein the concentration of the active ingredient is at least 100 mg/mL.

2. The stable liquid eutectic system according to claim 1, said system consisting of :

   - an active ingredient chosen from catechins,
   - a choline salt,
   - a polar protic solvent or aprotic polar solvent which is pharmaceutically, dermo-cosmetically or nutraceutically acceptable,

   wherein the molar ratio between the active ingredient, the choline salt, and the solvent is 0.25-1.5:1:5-50. wherein the concentration of the active ingredient is at least 100 mg/mL.

3. The stable liquid eutectic system according to claim 1 or 2, wherein the active ingredient is green tea catechins, in particularly chosen from epicatechins, epigallocatechin, epicatechin-3-gallate, epigallocatechin gallate, or a mixture thereof.

4. The stable liquid eutectic system according to any one of claims 1 to 3, wherein the choline salt is chosen from choline chloride, choline hydroxide, choline tartrate, choline dihydrogen citrate, choline acetate, and choline sulphate.

5. The stable liquid eutectic system according to any one of claims 1 to 4, wherein the solvent is an aprotic solvent chosen from the classes of solvents of sulfoxides, ketones, esters, amides, in particular dimethyl sulfoxide, dimethylacetamide, or a polar protic solvent chosen from water, ethanol, propylene glycol, glycerol.

6. The stable liquid eutectic system according to any one of claims 1 to 5, wherein said system is epigallocatechin gallate - choline chloride or choline tartrate - an aprotic polar solvent in a molar ratio of 1.0-1.5:1:6-10, preferably in a molar ratio of 1.0-1.3:1:6-10.

7. The stable liquid eutectic system according to any one of claims 1 to 4, wherein said system is epigallocatechin gallate - choline chloride or choline tartrate - a polar protic solvent in a molar ratio of 0.25-1:1:15-50.

8. A pharmaceutical, dermo-cosmetic or nutraceutical composition, comprising a stable liquid eutectic system as defined according to any one of claims 1 to 7, and a pharmaceutically, dermo-cosmetically or nutraceutically acceptable carrier.

9. The pharmaceutical, dermo-cosmetic or nutraceutical composition according to claim 8, comprising:

   - 0.1 % -15.0 % wt, compared to total weight of composition, of a eutectic system as defined according to any one of claims 1 to 6, and further
   - a pharmaceutically, dermo-cosmetically or nutraceutically acceptable polar aprotic co-solvent or polar protic co-solvent.

10. The pharmaceutical, dermo-cosmetic or nutraceutical composition according to claim 9, comprising:

    - 0.1 % -15.0 % wt, compared to total weight of composition, of a eutectic system as defined according to any one of claims 1 to 6, and further
    - 0.1-1.0 mmol/l of at least one chelating agent,
    - water or water saline buffer,
    - optionally at least one another antioxidant,

    wherein said solution is at a pH in a range of 2.0-6.0, preferably 3.0-5.0.

11. The pharmaceutical, dermo-cosmetic or nutraceutical composition according to claim 8-10, said composition further comprises vitamin C.

12. The pharmaceutical composition according to any one of claims 8-11, said composition is formulated in a self-emulsifying drug-delivery system, a self-micro emulsifying drug-delivery system or an oil-free system.

13. A pharmaceutical composition according to any one of claims 8 to 12, for its use as a medicament in the treatment of cancers, cardiovascular disorder, diabetes, neurodegenerative diseases, or dermatological diseases.

14. A dermo-cosmetic composition according to any one of claims 8 to 11, said composition is formulated to be a semi-solid or pasty topical composition.

15. An oral care product's composition according to any one of claims 8 to 11, said composition is formulated to be a liquid, semi-solid or pasty topical composition.

16. A unit dose formulated with the stable liquid eutectic system according to any one of claims 1 to 7, or of the pharmaceutical, or dermo-cosmetic composition or a nutraceutical composition according to any one of claims 8-15.

17. A food or a drink comprising a stable liquid eutectic system according to any one of claims 1-7, or a nutraceutical composition according to any one of claims 8-11, said food or drink comprises at least 10 mg of epigallocatechin gallate, from the eutectic system according to any one of claims 1 to 7.

18. The food or the drink according to claim 17, which is a product of a coffee capsule, or a tea capsule, or any type of ready-to-use powder concentrates to be dissolved in water or other beverages, said product comprising at least 10 mg of epigallocatechin gallate per gram of product, from the eutectic system according to any one of claims 1 to 7.

**Patentansprüche**

1. Ein stabiles, flüssiges, eutektisches System, umfassend:

   - einem Wirkstoff, ausgewählt aus Catechinen,
   - einem Cholinsalz,
   - einem polaren protischen Lösungsmittel oder aprotischen polaren Lösungsmittel, das pharmazeutisch, dermokosmetisch oder ernährungsphysiologisch akzeptabel ist,

   wobei das molare Verhältnis zwischen dem Wirkstoff, dem Cholinsalz und dem Lösungsmittel 0,25-3,5:1:5-75 ist, wobei die Konzentration des Wirkstoffs mindestens 100 mg/ml beträgt.

2. Das stabile, flüssige, eutektische System nach Anspruch 1, wobei das System besteht aus:

   - einem Wirkstoff, ausgewählt aus Catechinen,
   - einem Cholinsalz,
   - einem polaren protischen Lösungsmittel oder aprotischen polaren Lösungsmittel, das pharmazeutisch, dermokosmetisch oder ernährungsphysiologisch akzeptabel ist,

   wobei das molare Verhältnis zwischen dem Wirkstoff, dem Cholinsalz und dem Lösungsmittel 0,25-1,5:1:5-50 ist, wobei die Konzentration des Wirkstoffs mindestens 100 mg/ml beträgt.

3. Das stabile flüssige eutektische System nach Anspruch 1 oder 2, wobei der Wirkstoff Grüntee-Catechine sind, insbesondere solche ausgewählt aus Epicatechinen, Epigallocatechin, Epicatechin-3-gallat, Epigallocatechin-gallat, oder einer Mischung davon.

4. Das stabile flüssige eutektische System nach einem der Ansprüche 1 bis 3, wobei das Cholinsalz ausgewählt ist aus Cholinchlorid, Cholinhydroxid, Cholintartrat, Cholindihydrogencitrat, Cholinacetat und Cholinsulfat.

5. Das stabile flüssige eutektische System nach einem der Ansprüche 1 bis 4, wobei das Lösungsmittel ein aprotisches Lösungsmittel, ausgewählt aus den Lösungsmittelklassen der Sulfoxide, Ketone, Ester, Amide, insbesondere Dimethylsulfoxid, Dimethylacetamid, oder ein polares protisches Lösungsmittel, ausgewählt aus Wasser, Ethanol, Propylenglykol, Glycerin.

6. Das stabile flüssige eutektische System nach einem der Ansprüche 1 bis 5, wobei das System Epigallocatechingallat - Cholinchlorid oder Cholintartrat - ein aprotisches polares Lösungsmittel in einem molaren Verhältnis von 1,0-1,5:1:6-10, bevorzugt in einem molaren Verhältnis von 1,0-1,3:1:6-10 ist.

7. Das stabile flüssige eutektische System nach einem der Ansprüche 1 bis 4, wobei das System Epigallocatechingallat - Cholinchlorid oder Cholintartrat - ein polares protisches Lösungsmittel in einem molaren Verhältnis von 0,25-1:1:15-50 ist.

8. Pharmazeutische, dermo-kosmetische oder ernährungsphysiologische Zusammensetzung, umfassend ein stabiles flüssiges eutektisches System, wie in einem der Ansprüche 1 bis 7 definiert, und einen pharmazeutisch, dermo-kosmetisch oder ernährungsphysiologisch akzeptablen Träger.

9. Die pharmazeutische, dermo-kosmetische oder ernährungsphysiologische Zusammensetzung nach Anspruch 8, umfassend:

- 0,1 bis 15,0 Gew.-%, bezogen auf das Gesamtgewicht der Zusammensetzung eines eutektischen Systems wie in einem der Ansprüche 1 bis 6 definiert, und weiterhin,
- ein pharmazeutisch, dermo-kosmetisch oder ernährungsphysiologisch akzeptables polares aprotisches Co-Lösungsmittel oder polares protisches Co-Lösungsmittel.

10. Die pharmazeutische, dermo-kosmetische oder ernährungsphysiologische Zusammensetzung nach Anspruch 9, umfassend:

- 0,1 bis 15,0 Gew.-%, bezogen auf das Gesamtgewicht der Zusammensetzung eines eutektischen Systems wie in einem der Ansprüche 1 bis 6 definiert, und weiterhin,
- 0,1-1,0 mmol/l mindestens eines Chelatbildners,
- Wasser oder wässriger Salzpuffer,
- gegebenenfalls mindestens ein weiteres Antioxidans,

wobei diese Lösung einen pH-Wert in einem Bereich von 2,0 bis 6,0, bevorzugt 3,0 bis 5,0, hat.

11. Pharmazeutische, dermo-kosmetische oder ernäherungsphysiologische Zusammensetzung nach Ansprüchen 8 - 10, wobei die Zusammensetzung außerdem Vitamin C enthält.

12. Die pharmazeutische Zusammensetzung nach einem der Ansprüche 8 - 11, wobei die Zusammensetzung formuliert ist in einem selbstemulgierenden Arzneimittelabgabesystem, einem selbstmikroemulgierenden Arzneimittelabgabesystem oder einem ölfreien System.

13. Die pharmazeutische Zusammensetzung nach einem der Ansprüche 8 bis 12, zur Verwendung als Medikament in der Behandlung von Krebs, kardiovaskulären Erkrankungen, Diabetes, neurodegenerativen Erkrankungen oder dermatologischen Erkrankungen.

14. Eine dermo-kosmetische Zusammensetzung nach einem der Ansprüche 8 bis 11, wobei die Zusammensetzung als eine halbfeste oder pastöse topische Zusammensetzung formuliert ist.

15. Eine Zusammensetzung eines Mundpflegemittels nach einem der Ansprüche 8 bis 11, wobei die Zusammensetzung als eine flüssige, halbfeste oder pastöse topische Zusammensetzung formuliert ist.

16. Einheitsdosis, formuliert mit dem stabilen flüssigen eutektischen System nach einem der Ansprüche 1 bis 7 oder der pharmazeutischen oder dermokosmetischen Zusammensetzung oder einer nach einem der Ansprüche 8-15.

17. Ein Nahrungsmittel oder ein Getränk, umfassend ein stabiles flüssiges eutektisches System nach einem der Ansprüche 1-7, oder eine ernährungsphysiologische Zusammensetzung nach einem der Ansprüche 8-11, wobei dieses Lebensmittel oder Getränk mindestens 10 mg an Epigallocatechingallat aus dem eutektischen System nach einem der Ansprüche 1 bis 7 umfasst.

18. Nahrungsmittel oder Getränk nach Anspruch 17, welches ein Produkt einer Kaffeekapsel oder einer Teekapsel oder einer Art von gebrauchsfertigen Pulverkonzentraten ist, das in Wasser oder anderen Getränken aufgelöst werden

kann, wobei dieses Produkt mindestens 10 mg an Epigallocatechingallat pro Gramm Produkt aus dem eutektischen System nach einem der Ansprüche 1 bis 7 umfasst.

## Revendications

**1.** Système eutectique liquide stable comprenant :

- un ingrédient actif choisi parmi des catéchines,
- un sel de choline,
- un solvant polaire protique ou un solvant polaire aprotique qui est acceptable sur le plan pharmaceutique, dermo-cosmétique ou nutraceutique,
où le rapport molaire entre l'ingrédient actif, le sel de choline et le solvant est 0,25-3,5:1:5-75.
où la concentration en l'ingrédient actif est au moins de 100 mg/ml.

**2.** Le système eutectique liquide stable selon la revendication 1, où ledit système consiste en :

- un ingrédient actif choisi parmi des catéchines,
- un sel de choline,
- un solvant polaire protique ou un solvant polaire aprotique qui est acceptable sur le plan pharmaceutique, dermo-cosmétique ou nutraceutique,

où le rapport molaire entre l'ingrédient actif, le sel de choline et le solvant est 0,25-1,5:1:5-50.
où la concentration en l'ingrédient actif est au moins de 100 mg/ml.

**3.** Le système eutectique liquide stable selon la revendication 1 ou 2, où l'ingrédient actif est des catéchines du thé vert, en particulier choisi parmi des épicatéchines, l'épigallocatéchine, le 3-gallate d'épicatéchine, le gallate d'épigallocatéchine, ou un de leur mélange.

**4.** Le système eutectique liquide stable selon l'une quelconque des revendications 1 à 3, où le sel de choline est choisi parmi le chlorure de choline, l'hydroxyde de choline, le tartrate de choline, le dihydrogénocitrate de choline, l'acétate de choline et le sulfate de choline.

**5.** Le système eutectique liquide stable selon l'une quelconque des revendications 1 à 4, où le solvant est un solvant aprotique choisi parmi les classes de solvants des sulfoxydes, cétones, esters, amides, en particulier le diméthylsulfoxyde, le diméthylacétamide, ou un solvant polaire protique choisi parmi l'eau, l'éthanol, le propylène glycol, le glycérol.

**6.** Le système eutectique liquide stable selon l'une quelconque des revendications 1 à 5, où ledit système est le gallate d'épigallocatéchine - le chlorure de choline ou le tartrate de choline - un solvant polaire aprotique dans un rapport molaire de 1,0-1,5:1:6-10, de préférence dans un rapport molaire de 1,0-1,3:1:6-10.

**7.** Le système eutectique liquide stable selon l'une quelconque des revendications 1 à 4, où ledit système est le gallate d'épigallocatéchine - le chlorure de choline ou le tartrate de choline - un solvant polaire protique dans un rapport molaire de 0,25-1:1:15-50.

**8.** Composition pharmaceutique, dermo-cosmétique ou nutraceutique, comprenant un système eutectique liquide stable tel que défini selon l'une quelconque des revendications 1 à 7, et un véhicule acceptable sur le plan pharmaceutique, dermo-cosmétique ou nutraceutique.

**9.** La composition pharmaceutique, dermo-cosmétique ou nutraceutique selon la revendication 8, comprenant :

- 0,1 % -15,0 % en poids, rapporté au poids total de la composition, d'un système eutectique tel que défini selon l'une quelconque des revendications 1 à 6, et en outre
- un co-solvant polaire aprotique ou un co-solvant polaire protique acceptable sur le plan pharmaceutique, dermo-cosmétique ou nutraceutique.

**10.** La composition pharmaceutique, dermo-cosmétique ou nutraceutique selon la revendication 9 comprenant :

- 0,1 % -15,0 % en poids, rapporté au poids total de la composition, d'un système eutectique tel que défini selon l'une quelconque des revendications 1 à 6, et en outre
- 0,1-1,0 mmol/l d'au moins un agent chélatant,
- de l'eau ou un tampon salin aqueux,
- éventuellement au moins un autre antioxydant,

où ladite solution est à un pH dans une plage de 2,0-6,0, préférentiellement 3,0-5,0.

**11.** La composition pharmaceutique, dermo-cosmétique ou nutraceutique selon la revendication 8-10, ladite composition comprend en outre une vitamine C.

**12.** La composition pharmaceutique selon l'une quelconque des revendications 8-11, ladite composition est formulée dans un système de libération de médicaments auto-émulsifiant, un système de libération de médicaments auto-microémulsifiant ou un système exempt d'huile.

**13.** Composition pharmaceutique selon l'une quelconque des revendications 8 à 12 pour son utilisation en tant que médicament dans le traitement de cancers, de désordres cardio-vasculaires, de diabètes, de maladies neurodégénératives ou de maladies dermatologiques.

**14.** Composition dermo-cosmétique selon l'une quelconque des revendications 8 à 11, ladite composition est formulée pour se présenter sous la forme d'une composition à usage topique semi-solide ou pâteuse.

**15.** Composition pour produit d'hygiène buccale selon l'une quelconque des revendications 8 à 11, ladite composition est formulée pour se présenter sous la forme d'une composition à usage topique liquide, semi-solide ou pâteuse.

**16.** Dose unitaire formulée avec le système eutectique liquide stable selon l'une quelconque des revendications 1 à 7 ou de la composition pharmaceutique ou dermo-cosmétique ou d'une composition nutraceutique selon l'une quelconque des revendications 8-15.

**17.** Denrée alimentaire ou boisson comprenant un système eutectique liquide stable selon l'une quelconque des revendications 1-7 ou une composition nutraceutique selon l'une quelconque des revendications 8-11, ladite denrée alimentaire ou boisson comprend au moins 10 mg de gallate d'épigallocatéchine, à partir du système eutectique selon l'une quelconque des revendications 1 à 7.

**18.** Denrée alimentaire ou boisson selon la revendication 17 qui est un produit consistant en une capsule de café ou une capsule de thé ou tout type de concentrés en poudre prêts à l'emploi à dissoudre dans de l'eau ou d'autres boissons, ledit produit comprenant au moins 10 mg de gallate d'épigallocatéchine par gramme de produit, à partir du système eutectique selon l'une quelconque des revendications 1 à 7.

Figure 1A

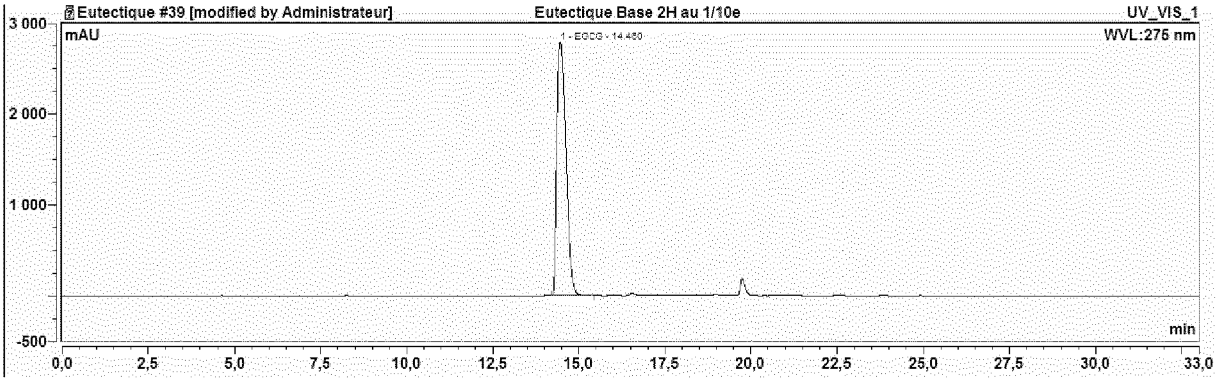


Figure 1B

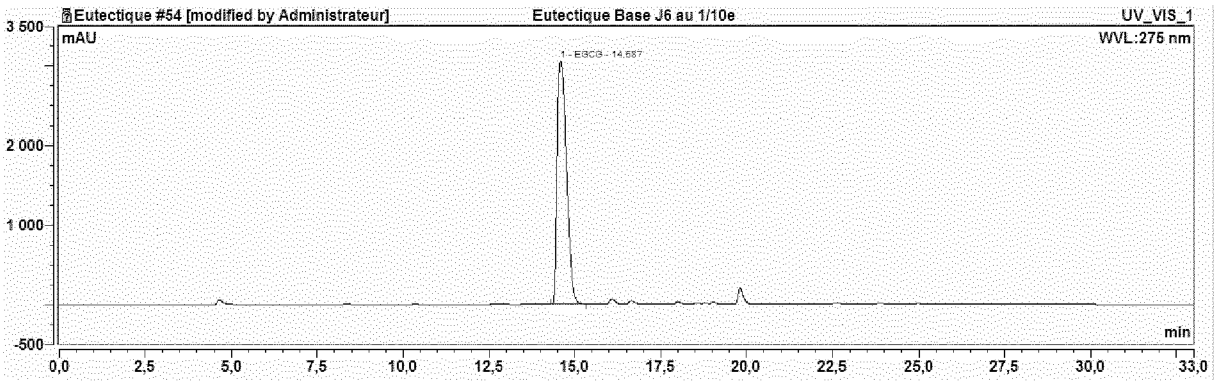


Figure 1C

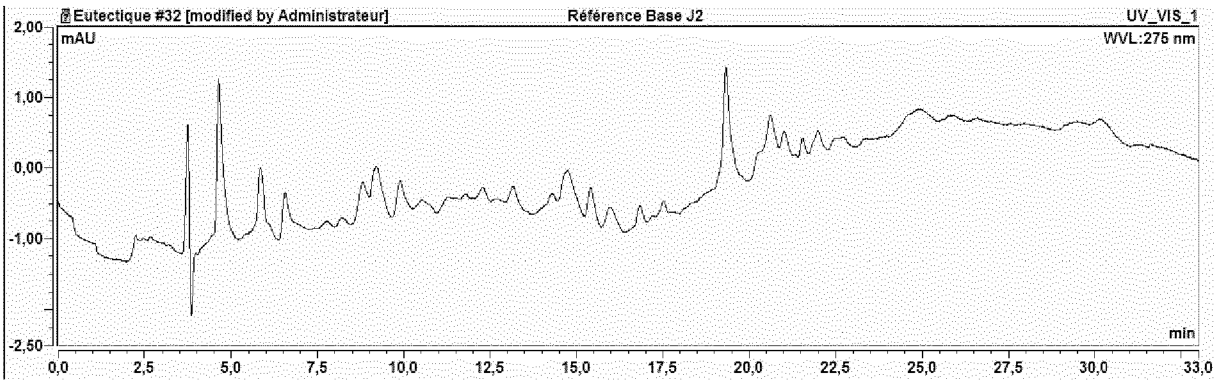

Figure 2A

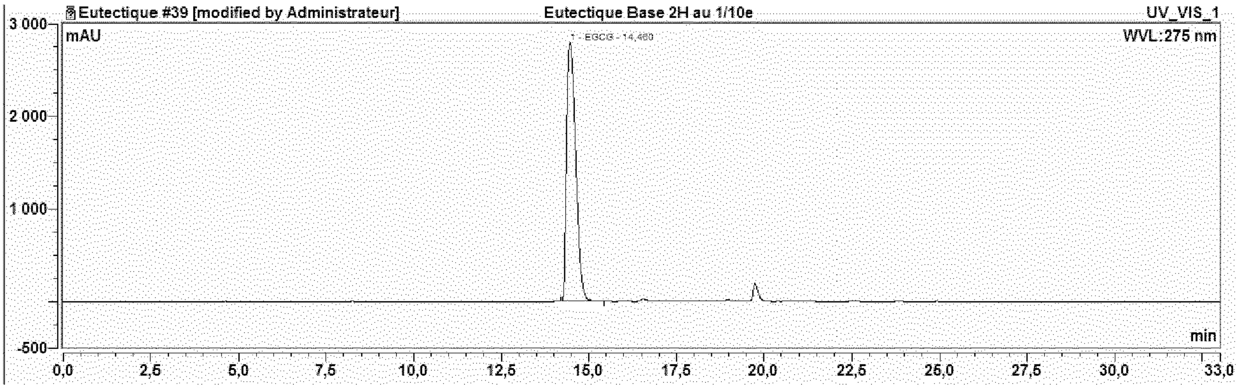


Figure 2B

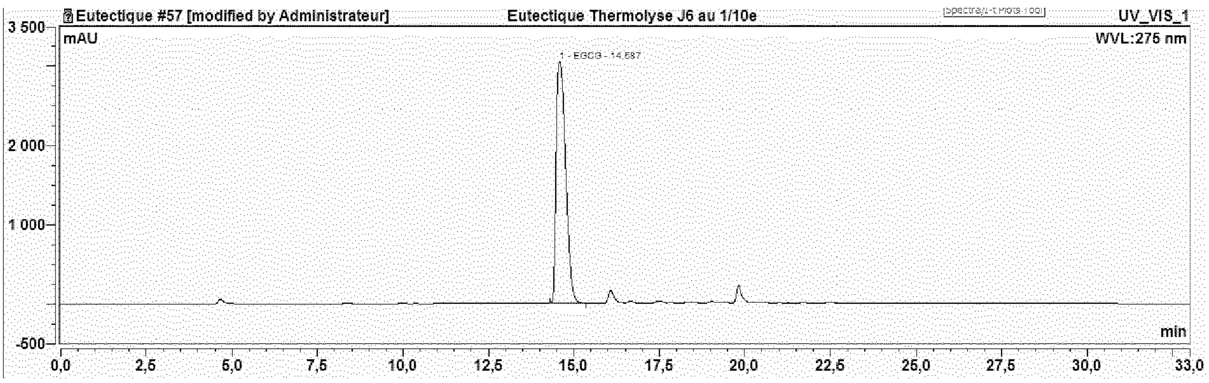


Figure 2C

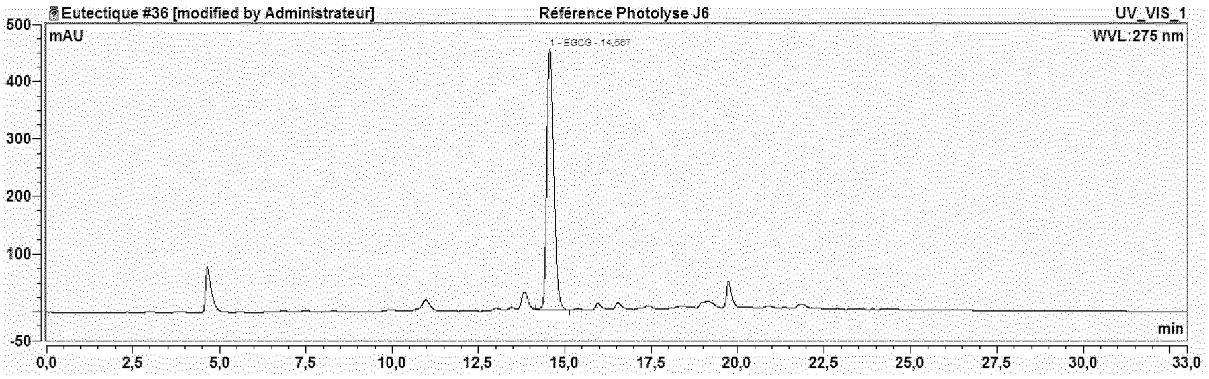

Figure 3A

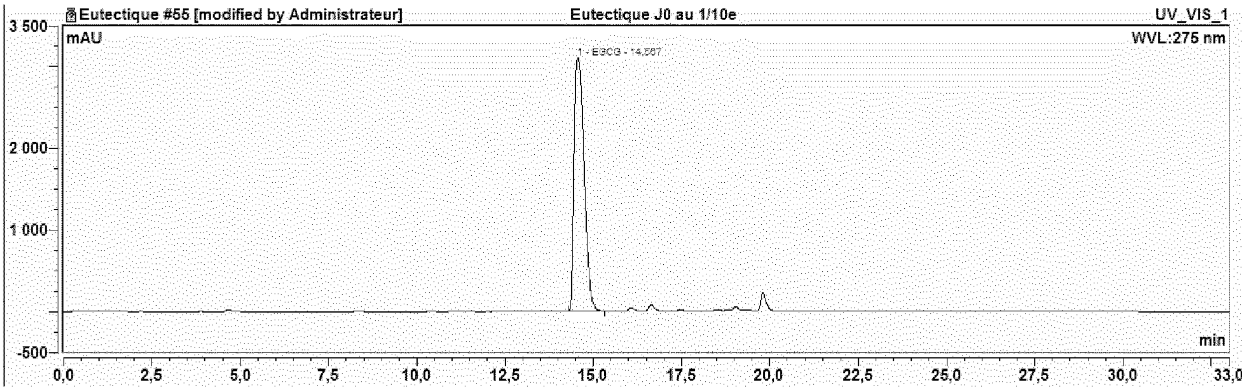


Figure 3B

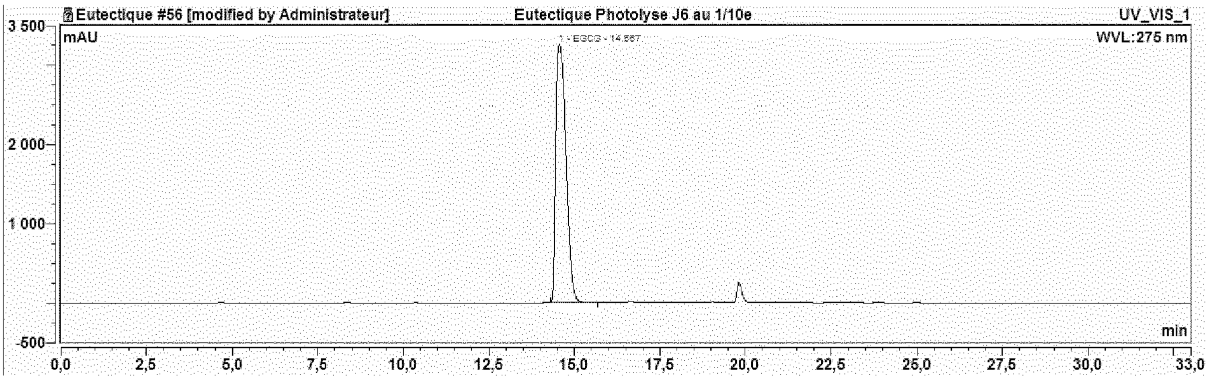


Figure 3C

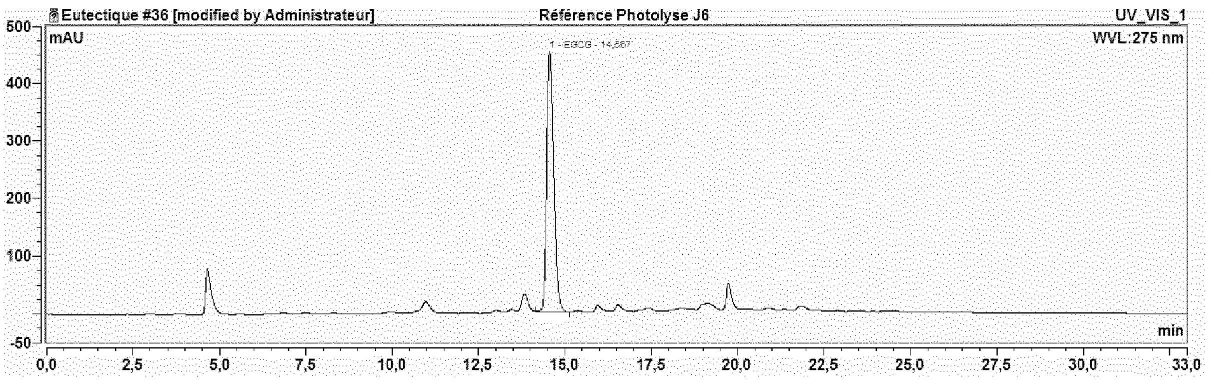

Figure 4A

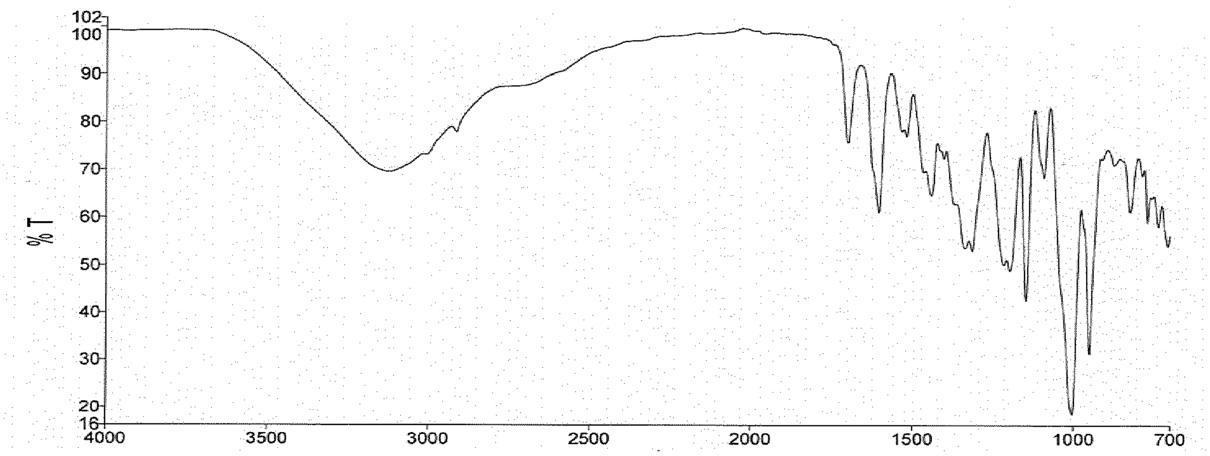


Figure 4B

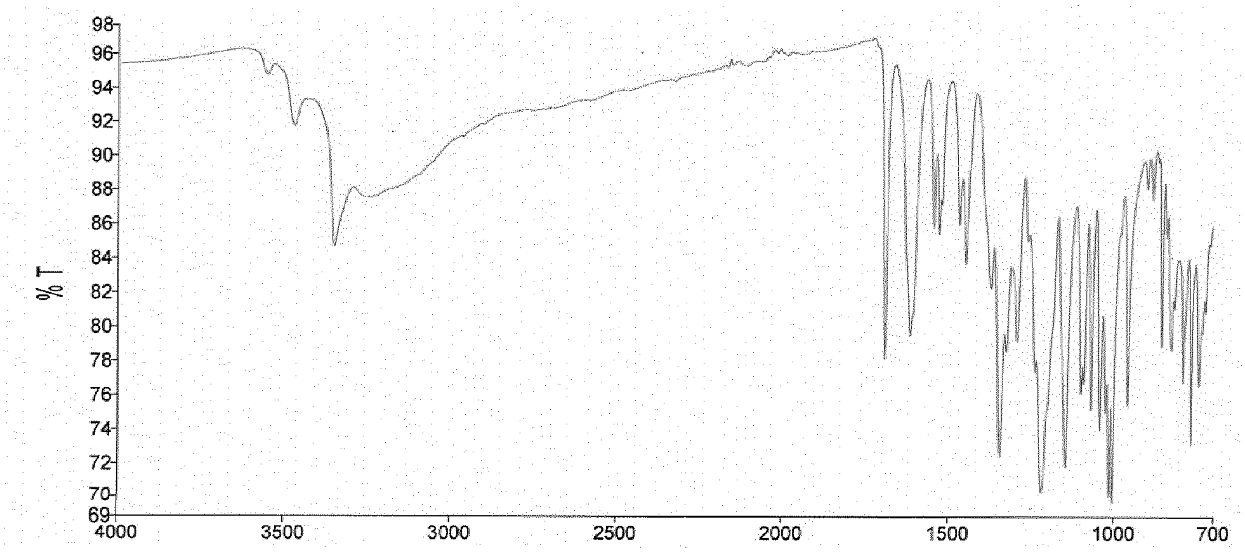

Figure 5

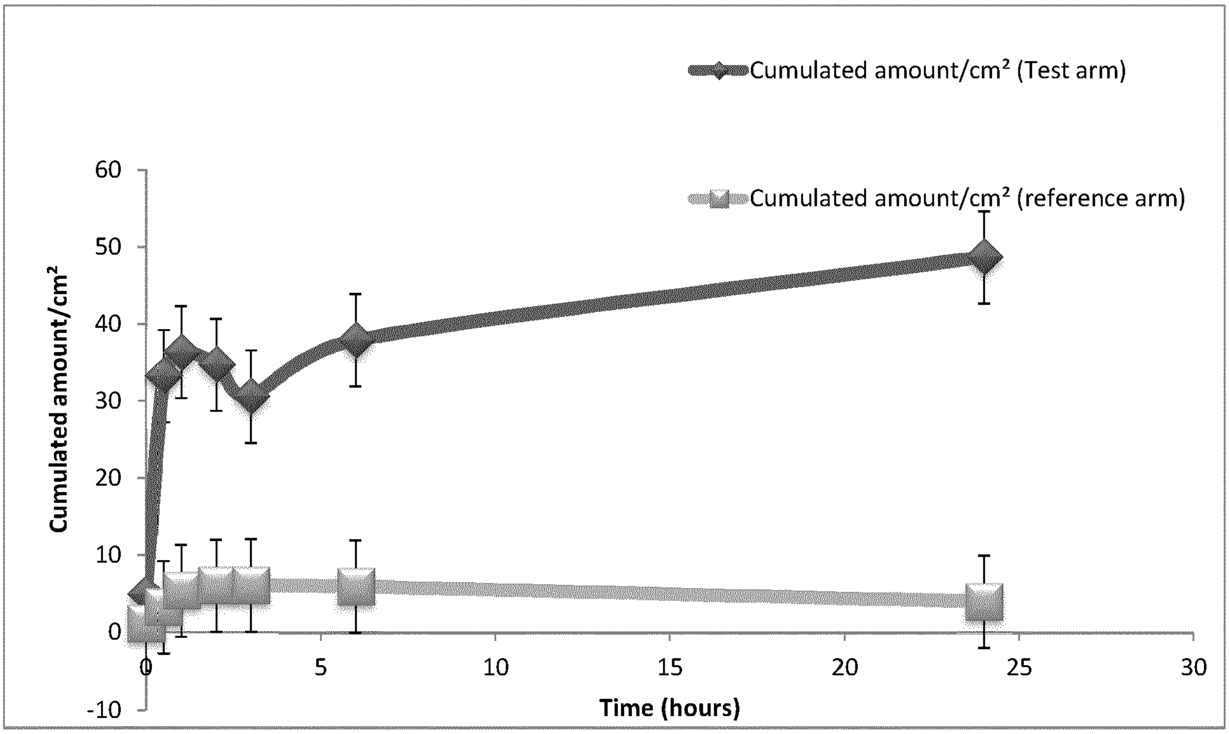


Figure 6

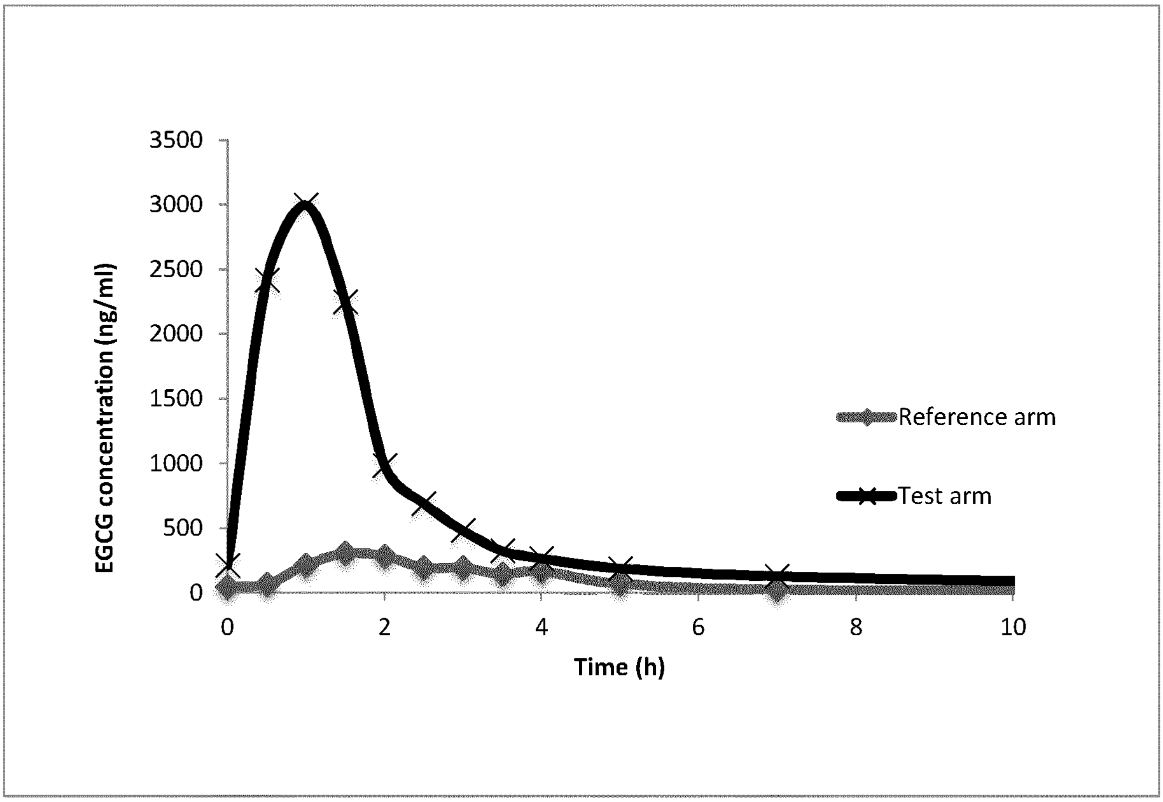

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20140088030 A
- EP 17306623 A
- WO 2014055830 A1


### Non-patent literature cited in the description


- *Crit. Rev. Food Sci. Nutr.,* 2015, vol. 55, 792-80
- *Curr. Opin. Clin. Nutr. Metab. Care,* 2008, vol. 11, 758-765
- *Geriatr. Gerontol. Int.,* 2014, vol. 14, 238-250
- *Int. J. Dent. Hyg.,* 2011, vol. 9, 110-116
- *ISRN Prev. Med.,* 2012, 975148
- *J. Agric. Food Chem.,* 2010, vol. 58, 1296-1304
- *Int. Res. J. Pharm.,* 2012, vol. 3, 139-148
- *Front. Microbiol.,* 2014, vol. 5, 434
- **CHOW et al.** *Clin Cancer Res,* 2005, vol. 11, 4627-4633
- **KRUPKOVA et al.** *Journal of Nutritional Biochemistry,* 2016, vol. 37, 1-12
- *J. Pharm. Biomed. Anal.,* 2011, vol. 56, 692-697
- *Chem. Pharm. Bull.,* 2014, vol. 62 (4), 328-335
- *Nutrients,* 2016, vol. 8, E307
- *Journal of Nutritional Biochemistry,* 2016, vol. 37, 1-12
- **LOURDES et al.** *J. Agric. Food Chem.,* 2007, vol. 55, 8857-8863